(19) Europäisches Patentamt
European Patent Office
Office européen des brevets

(11) EP 3 842 075 B1

(12) EUROPÄISCHE PATENTSCHRIFT

(45) Veröffentlichungstag und Bekanntmachung des Hinweises auf die Patenterteilung:
**07.06.2023 Patentblatt 2023/23**

(21) Anmeldenummer: **19219302.7**

(22) Anmeldetag: **23.12.2019**

(51) Internationale Patentklassifikation (IPC):
***A61L 15/26*** *(2006.01)* ***A61L 15/42*** *(2006.01)*

(52) Gemeinsame Patentklassifikation (CPC):
(C-Sets verfügbar)
**A61L 15/425; A61L 15/26** (Forts.)

(54) **SILOXAN ENTHALTENDE WUNDAUFLAGE**

SILOXANE-CONTAINING WOUND DRESSING

PANSEMENT CONTENANT DU SILOXANE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**30.06.2021 Patentblatt 2021/26**

(73) Patentinhaber: **Paul Hartmann AG**
**89522 Heidenheim (DE)**

(72) Erfinder:
- **Croizat, Pierre**
  **89542 Herbrechtingen (DE)**
- **Deibler, Martin**
  **89542 Herbrechtingen (DE)**
- **Eckstein, Axel**
  **89522 Heidenheim (DE)**

(74) Vertreter: **Ter Meer Steinmeister & Partner**
**Patentanwälte mbB**
**Nymphenburger Straße 4**
**80335 München (DE)**

(56) Entgegenhaltungen:
**EP-A1- 3 106 181**
**WO-A1-2009/007018**
**WO-A1-2016/156619**
**DE-A1-102008 031 182**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

(52) Gemeinsame Patentklassifikation (CPC): (Forts.)

C-Sets
**A61L 15/26, C08L 75/04, C08L 83/04**

## Beschreibung

Die Erfindung betrifft eine Wundauflage, welche bevorzugt zur Wundbehandlung im feuchten oder feuchtnassen Milieu eingesetzt werden kann. Die Wundauflage umfasst hierbei (a) eine Wundkontaktschicht umfassend einen Siloxan-enthaltenden Polyurethanschaumstoff, ein Siloxan-funktionalisiertes Polyurethan und (b) eine hydrophile Schicht umfassend einen hydrophilen Schaumstoff. Die Wundauflage ist bevorzugt modifizierbar, d.h. an die Wunde anpassbar.

### Hintergrund der Erfindung

Unter einer Wunde wird die Trennung des Zusammenhangs von Geweben der Körperhülle bei Menschen oder Tieren verstanden. Sie kann mit einem Verlust an Substanz verbunden sein.

Verbandsmaterialien wie Wundauflagen mit aktivierten, Flüssigkeit-enthaltenden Wundkissen sind bei der Behandlung von chronischen Wunden bekannt. Die Kissen weisen meist einen hydrophilen Kern auf, der von einem stabilisierenden Trägermaterial umhüllt ist. WO 2016/156619 A1 beschreibt ein derartiges Produkt und ein entsprechendes im Markt befindliches Beispiel einer solchen Wundauflage stellt HydroClean (Paul Hartmann AG) dar. Das Produkt enthält ein Wundkissen, das einen in Cellulose- und Zellstofffasern eingebetteten Saug-/Spülkörper umfasst, wobei der Saug-/Spülkörper superabsorbierende Polyacrylat-Partikel (SAP) enthält. Zudem wird das Wundkissen beidseitig mit Ringerlösung beaufschlagt bzw. aktiviert. Wundfern ist das flüssigkeitsenthaltende Wundkissen, das auch als eine flüssigkeitsenthaltende Matrix betrachtet werden kann, durch eine wasserundurchlässige Polypropylenfolie abgekapselt. Wundnah interagiert die aus Polypropylenstrick (PP) bestehende Wundkontaktschicht mit dem durch das Produkt befeuchteten Wundgrund, indem Wundbeläge (Fibrin, entzündliche, infizierte Ablagerungen) angelöst und diese durch Interaktion mit dem PP-Material entfernt werden. Das Produkt fördert neben dem Debridement-Effekt die Wundheilung durch das Binden von heilungshemmenden Proteinen (z.B. Matrix-Metalloproteasen) in der SAP-Matrix. Um übermäßige Adhäsion der Wundauflage mit dem Wundgrund zu vermeiden, sind auf dem Produkt Silikonstreifen lokal aufgetragen. Um eine Wundkontaminationen durch die pulverförmigen, superabsorbierenden Polyacrylat-Partikel zu vermeiden, müssen diese in dem Wundkissen gebunden werden. So werden die superabsorbierenden Partikel in einem kissenartigen Verbund eingebracht, der durch Ringerlösung aktiviert wird. Derartige Wundkissen umfassende Wundauflagen werden dann in fertigen, vorgegebenen Größen vertrieben.

Diese Flüssigkeit-enthaltenden Wundauflagen sind jedoch in ihrer Größe nicht anpassbar, da beispielsweise beim Zuschneiden auf die Wundgeometrie die Hülle des Wundkissens beschädigt und somit die Wunde durch austretende SAP-Partikel kontaminiert würde. Damit werden bei den kommerziell erhältlichen Produkten eine standardisierte Wundgeometrie und Anwendungssituation zum erfolgreichen Einsatz vorausgesetzt. Weiterhin ist das Verkleben mit dem Wundgrund bei der oben beschriebenen Wundauflage stark vom Hydratisierungsgrad des Verbandes abhängig und auf die einseitig, lokal aufgetragenen Silikon-Streifen begrenzt.

Der vorliegenden Erfindung liegt die Aufgabe zugrunde, die Behandlung von Wunden weiter zu verbessern und die Nachteile des Standes der Technik zu überwinden. Hierzu soll durch die vorliegende Erfindung insbesondere eine Wundauflage bereitgestellt werden, die flexibel eingesetzt werden kann und mit der eine möglichst wirksame und schonende Wundbehandlung erreicht werden kann.

Dazu soll eine Wundauflage, bevorzugt für die feuchte Wundbehandlung, bereitgestellt werden, die auf den Wundsitus anpassbar ist. Insbesondere soll diese Anpassbarkeit auf den jeweiligen Wundsitus unter Beibehaltung der Fähigkeit inhibitorische Komponenten wie Fibrin und entzündliche Ablagerungen aus dem Wundgrund abzuleiten erzielt werden.

Weiterhin liegt der vorliegenden Erfindung die Aufgabe zugrunde, eine Wundauflage bereitzustellen, bei der das Risiko von Mazerationen verringert werden kann. Zudem soll ein Verkleben der Wundauflage mit dem Wundgrund vermieden werden.

Außerdem soll eine Wundauflage bereitgestellt werden, die sich durch ein vorteilhaftes Debridement auszeichnet.

Die DE102008031182 beschreibt mehrschichtige Wundauflagen für die feuchte Wundbehandlung, mit einer ersten Schicht enthaltend eine Hydrogelmatrix und einer zweiten Schicht enthaltend einen hydrophilen Polymerschaum.

### Zusammenfassung der Erfindung

Vorstehend genannte Aufgaben können erfindungsgemäß durch eine Wundauflage gelöst werden, welche eine Wundkontaktschicht, die einen Siloxan-enthaltenden Polyurethanschaumstoff oder ein Siloxan-funktionalisiertes Polyurethan umfasst, und eine hydrophile Schicht, die einen hydrophilen Schaumstoff umfasst, enthält.

Ebenfalls ein Gegenstand der Erfindung ist ein Verfahren zur Herstellung der erfindungsgemäßen Wundauflage, umfassend das Verbinden der Wundkontaktschicht (a) mit der hydrophilen Schicht (b), wobei das Verbinden der Schichten vorzugsweise Verkleben mit einem Klebstoff, Ultraschallschweißen in einem kontinuierlichen Laminierprozess oder

einen Thermobonding-Prozess umfasst.

Ein weiterer Gegenstand der Erfindung ist eine pharmazeutisch verträgliche Lösung, insbesondere Ringerlösung, zur Verwendung in der Behandlung von Wunden, insbesondere in der Granulationsphase, wobei die pharmazeutisch verträgliche Lösung in einer hydrophilen Schicht der erfindungsgemäßen Wundauflage enthalten ist, wobei die Wundauflage umfasst

(a) Wundkontaktschicht umfassend einen Siloxan-enthaltenden Polyurethanschaumstoff oder ein Siloxan-funktionalisiertes Polyurethan,
(b) hydrophile Schicht umfassend einen hydrophilen Schaumstoff.

Ein Vorteil der erfindungsgemäßen Wundauflage besteht darin, dass ein Verkleben und/oder Verwachsen des Wundgrundes mit der Wundauflage selbst über einen Zeitraum von beispielsweise bis zu 3 Tagen weitgehend vermieden werden kann. Eine Traumatisierung der Wunde beim Verbandswechsel kann vermieden werden. Dies erhöht die Wirksamkeit der Wundbehandlung. Somit wird beim Patienten ein Wechsel der Wundauflagen in Intervallen von beispielsweise bis zu drei Tagen ermöglicht. Während des Intervalls von drei Tagen wird die erfindungsgemäße Wundauflage vom Patienten als angenehm empfunden. Druckreiz, Juckreiz und Hautrötungen werden in der Regel vermieden. Beim Wechsel der Wundauflage nach einem Zeitraum von 3 Tagen treten wenig unangenehme Gerüche auf. Die Keimdichte in der ausgewechselten Wundauflage ist unerwartet niedrig.

Weiterhin ist die erfindungsgemäße Wundauflage dem Wundsitus anpassbar. Das bedeutet, dass die vorliegende Wundauflage mit einem gebräuchlichen Schneidewerkzeug wie beispielsweise einer Schere auf eine dem Wundsitus anpassbare Größe und Form zurechtgeschnitten werden kann, wobei ihre Fähigkeit, inhibitorische Komponenten wie Fibrin und entzündliche Ablagerungen aus dem Wundgrund abzuleiten, weiterhin erhalten bleibt. Das bedeutet, dass die erfindungsgemäße Wundauflage selbst nach dem Zurechtschneiden keine schädlichen Partikel freisetzt und/oder ihre positiven Eigenschaften nicht beeinträchtigt werden. Zusätzlich können sowohl Mazeration und/oder ein Verkleben der Wundauflage mit dem Wundgrund verringert oder bevorzugt vermieden werden und/oder ein vorteilhaftes Debridement erreicht werden.

Detaillierte Beschreibung der Erfindung

Die Erfindung betrifft eine Wundauflage, welche bevorzugt zur Wundbehandlung im feuchten oder feuchtnassen Milieu eingesetzt werden kann. Die Wundauflage umfasst hierbei (a) eine Wundkontaktschicht, welche einen Siloxan-enthaltenden Polyurethanschaumstoff oder ein Siloxan-funktionalisiertes Polyurethan umfasst und (b) eine einen hydrophilen Schaumstoff umfassende hydrophile Schicht.

Die Bestandteile (a) und (b) der erfindungsgemäßen Wundauflage werden nachstehend beschrieben.

Die erfindungsgemäße Vorrichtung umfasst eine Wundkontaktschicht (a), welche einen Siloxan-enthaltenden Polyurethanschaumstoff oder einen Siloxan-funktionalisierten Polyurethanschaum umfasst. Über diese Wundkontaktschicht wird die vorliegende Wundauflage mit der Wunde in Kontakt gebracht, d.h. zwischen der Wunde und der Wundkontaktschicht befinden sich keine weiteren Schichten/Auflagen oder ähnliches.

Der von der Wundkontaktschicht umfasste Siloxan-enthaltende Polyurethanschaumstoff oder Siloxan-funktionalisierte Polyurethanschaum, welcher in der erfindungsgemäßen Wundauflage Verwendung findet, wird nachstehend genauer beschrieben.

Allgemein wird unter einem Polyurethan ein Polymer verstanden, welches Urethangruppen, -O-C(O)-NH-, enthält. Im Fall des vorliegenden Siloxan-enthaltenden Polyurethans enthält das Polyurethan mindestens zwischen zwei Urethangruppen eine Polysiloxan-Verbindung. Unter Polysiloxanen werden synthetische Polymere verstanden, bei denen Siliziumatome über Sauerstoffatome verknüpft sind.

Als Schaumstoffe werden üblicherweise Werkstoffe mit über die gesamte Masse verteilten Zellen (offen, geschlossen oder beides) verstanden. Werkstoffe weisen üblicherweise somit eine Rohdichte (gemäß DIN EN ISO 845) auf, die niedriger ist als die Dichte der Gerüstsubstanz.

In einer bevorzugten Ausführungsform ist der Siloxan-enthaltende Polyurethanschaumstoff ein offenzelliger, quervernetzter Siloxan-enthaltender Polyurethanschaumstoff.

Eine Zelle ist der bei der Herstellung von Schaumstoffen gebildete einzelne Hohlraum, der von Zellwänden und/oder Zellstegen teilweise oder vollständig umschlossen ist. Eine offene Zelle ist üblicherweise eine Zelle, die über die Gasphase mit anderen Zellen in Verbindung steht. Im Rahmen dieser Anmeldung bedeutet der Begriff offenzellig, dass im Siloxan-enthaltende Polyurethanschaumstoff mindestens 60 % offene Zellen, bevorzugt mindestens 90 % offene Zellen, mehr bevorzugt mindestens 98 % offene Zellen, insbesondere im Wesentlichen 100 % offene Zellen, bezogen auf die Gesamtzahl an Zellen, vorhanden sind.

Unter "quervernetzt" wird üblicherweise verstanden, dass lineare Polymerketten über kovalente Bindungen quer (d.h. nicht am Ende der linearen Ketten) miteinander verbunden sind.

Erfindungsgemäß ist der Siloxan-enthaltende Polyurethanschaumstoff erhältlich durch Umsetzung einer härtbaren Mischung umfassend die Komponenten:

(i) Polyisocyanat

(ii) Siloxan der Formel (A)

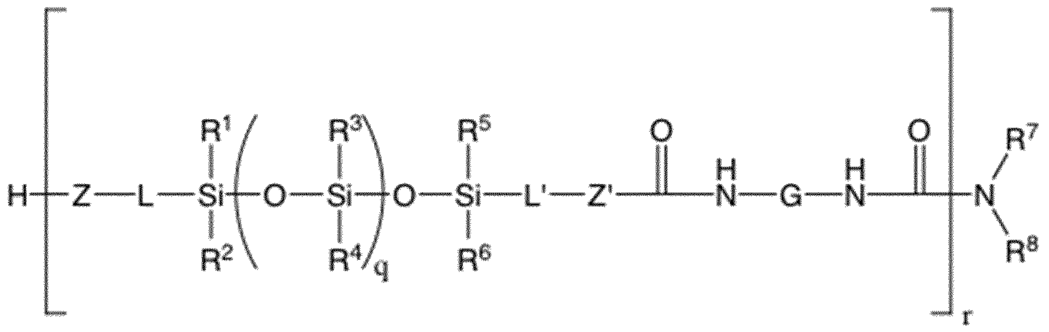

Formel (A)

wobei

$R^1$, $R^2$, $R^3$, $R^4$, $R^5$ und $R^6$ unabhängig voneinander Wasserstoff, ein substituierter oder ein unsubstituierter aliphatischer Kohlenwasserstoffrest mit 1 bis 8 C-Atomen oder ein substituierter oder ein unsubstituierter aromatischer Kohlenwasserstoffrest mit 6 bis 12 C-Atomen sind,
$R^7$ und $R^8$ unabhängig voneinander Wasserstoff, ein substituierter oder ein unsubstituierter aliphatischer Kohlenwasserstoffrest mit 1 bis 8 C-Atomen oder ein substituierter oder ein unsubstituierter aromatischer Kohlenwasserstoffrest mit 6 bis 12 C-Atomen sind, unter der Voraussetzung, dass mindestens einer der Reste $R^7$ und $R^8$ mindestens eine Hydroxyl- oder Thiolgruppe aufweist,
Z und Z' unabhängig voneinander Sauerstoff (O) oder Schwefel (S) sind,
L und L' unabhängig voneinander ein substituierter oder ein unsubstituierter aliphatischer Kohlenwasserstoffrest mit 1 bis 6 C-Atomen, wobei eines des C-Atome davon durch Sauerstoff (O) ersetzt sein kann, sodass eine Etherbindung vorliegt,
oder ein substituierter oder unsubstituierter aromatischer Kohlenwasserstoffrest mit 6 bis 12 C-Atomen ist, welche ein Heteroatom enthalten können,
G ein substituierter oder ein unsubstituierter aliphatischer Kohlenwasserstoffrest mit 1 bis 6 C-Atomen, oder ein substituierter oder unsubstituierter aromatischer Kohlenwasserstoffrest mit 6 bis 12 C-Atomen ist, welche ein Heteroatom enthalten können,
q eine ganze Zahl von 1 bis 100 ist,
r eine ganze Zahl von 1 bis 10 ist.

In einer bevorzugten Ausführungsform können die Polyisocyanate (i) Verbindungen mit mindestens 2 bis 8 Isocycanatgruppen, bevorzugt 2 bis 4 Isocycanatgruppen, insbesondere 2 oder 3 Isocycanatgruppen, sein.
Beispiele hierfür sind Diisocyanate wie 1,6-Hexamethylendiisocyant (HDI); 1-Iso-cyanato-3-isocyanatomethyl-3,5,5-trimethylcyclohexan (Isophorondiisocyanat, IPDI); 4,4'-Diisocyanatodicyclohexyl-methan ($H_{12}$MDI); 2,4-Toluoldiisocyanat (2,4-TDI); 2,6-Toluoldisiocynat (2,6-TDI); 2,2'-Diphenylmethandisocyanat (2,2'-MDI), 2,4'-Diphenylmethandisocyanat (2,4'-MDI), 4,4'-Diphenylmethandisocyanat (4,4'-MDI), Naphthalin-1,5-diisocyanat (NDI); 1,3-Bis(1-isocyanato-1-methylethyl)-benzol (TMXDI), Triisocyanate wie 4,4',4"-Triphenylmethantriisocyanat, oder Polyisocyanate wie polymeres MDI (p-MDI) sowie Gemische davon.
Bevorzugt werden die Polyisocyanate (i) in einer Menge von 0,1 bis 150 Gew.-%, bevorzugt von 1 bis 100 Gew.-%, insbesondere von 10 bis 50 Gew.-% eingesetzt, bezogen auf das Gewicht des Siloxans (ii).
Ein aliphatischer Kohlenwasserstoffrest mit 1 bis 8 C-Atomen kann eine Alkylgruppe mit 1 bis 8 C-Atomen, eine Cycloalkygruppe mit 3 bis 8 C-Atomen, eine Alkenylgruppe mit 2 bis 8 C-Atomen, eine Cycloalkenylgruppe mit 3 bis 8 C-Atomen sowie eine Alkinylgruppe mit 2 bis 8 C-Atomen sein.
Beispiele für Alkylgruppen mit 1 bis 8 C-Atomen sind Methyl-, Ethyl-, Propyl-, Isopropyl-, Butyl-, Isobutyl-, tert. Butyl-, Pentyl-, Isopentyl-, neo-Pentyl-, Hexyl-, Isohexyl-, Heptyl- und Oktylgruppen.
Beispiele für Cycloalkylgruppen mit 3 bis 8 C-Atomen sind Cyclopropyl-, Cyclobutyl-, Cyclopentyl-, Cyclohexyl-,

Cyclohepyl- und Cyclooctylgruppen.

Beispiele für Alkenylgruppen mit 2 bis 8 C-Atomen sind Vinyl-, Allyl-, 3-Methyl-2-Butenyl-Gruppen.

Beispiele für Cycloalkenylgruppen mit 3 bis 8 C-Atomen sind Cyclopropenyl-, Cyclopentenyl-, Cyclohexenyl- und Cyclooctenylgruppen.

Beispiele für Alkinylgruppen mit 2 bis 8 C-Atomen sind Ethinyl-, 1-Propinyl- und 2-Propinylgruppen.

Beispiele für einen aromatischen Kohlenwasserstoffrest mit 6 bis 12 C-Atomen sind Benzol-, Indan-, Naphthalin- und 1,2,3,4-Tetrahydronaphthalingruppen.

Der aliphatische Kohlenwasserstoffrest mit 1 bis 8 C-Atomen und der aromatische Kohlenwasserstoffrest mit 6 bis 12 C-Atomen können substituiert sein und einen oder mehrere Substituenten tragen.

Beispiele für Substituenten sind Halogenide, Nitro, Cyano, Carbonsäuren, Carbonsäureester, Carbonsäureamide, Hydroxy, optional geschütztes Hydroxy, Amin, optional geschütztes Amin, Monoalkylamin, optional geschütztes Monalkylamin, Dialkylamin, Alkylgruppen mit 1 bis 6 C-Atomen, Alkoxygruppen mit 1 bis 6 C-Atomen, Aryloxygruppen mit 6 bis 12 C-Atomen.

Schutzgruppen für Amine sind beispielsweise Boc (tert-Butyloxycarbonyl), Z oder Cbz (Benzyloxycarbonyl), Benzyl, Benzhydryl und Fmoc (Fluorenylmethylenoxycarbonyl).

Schutzgruppen für Hydroxygruppen sind beispielsweise Ester, wie Benzoesäure- oder Pivalinsäureester, und Silylether wie Trimethylsilylether, Triethylsilylether, tert-Butyldimethylsilylether und tert-Butyldiphenylsilylether.

In einer bevorzugten Ausführungsform sind jeweils $R^1$ und $R^2$, $R^3$ und R4 sowie $R^5$ und $R^6$ identische Reste, bevorzugt aliphatische Reste mit 1 bis 6 C-Atomen.

Besonders bevorzugt sind $R^1$, $R^2$, $R^3$, $R^4$, $R^5$ und $R^6$ identische Reste, mehr bevorzugt aliphatische Reste mit 1 bis 6 C-Atomen, noch mehr bevorzugt aliphatische Reste mit 1 oder 2 C-Atomen, insbesondere Methyl.

Alternativ besonders bevorzugt sind $R^1$, $R^2$, $R^3$, $R^4$, $R^5$ und $R^6$ identische Reste, mehr bevorzugt aromatische Reste mit 6 bis 12 C-Atomen, insbesondere Phenyl.

In einer bevorzugten Ausführungsform können $R^7$ und $R^8$ identische Reste sein und jeweils eine Hydroxygruppe aufweisen. Besonders bevorzugt sind $R^7$ und $R^8$ eine Hydroxylalkylgruppe mit 1 bis 4 C-Atomen, insbesondere Hydroxyethyl.

Z und Z' können vorzugsweise identisch sein. Besonders bevorzugt sind Z und Z' jeweils Sauerstoff (O).

In einer bevorzugten Ausführungsform sind L und L' unabhängig voneinander ein substituierter oder ein unsubstituierter aliphatischer Kohlenwasserstoffrest mit 1 bis 6 C-Atomen, wobei eines des C-Atome davon durch Sauerstoff (O) ersetzt sein kann, sodass eine Etherbindung vorliegt.

Weiter ist bevorzugt, dass L und L' identisch sind.

Besonders bevorzugt sind L und L' jeweils ein unsubstituierter aliphatischer Kohlenwasserstoffrest mit 1 bis 4 C-Atomen, wobei eines der C-Atome davon durch Sauerstoff (O) ersetzt sein kann, sodass eine Etherbindung vorliegt. Insbesondere sind L und L' $-CH_2-$ (Methylen) oder $-CH_2-O-CH_2-CH_2-$ (Ethoxymethylen), im speziellen Methylen.

In einer bevorzugten Ausführungsform ist G ein substituierter aromatischer Kohlenwasserstoffrest mit 6 bis 13 C-Atomen. Beispiele für G sind folgende: Phenylen, 2,4-Toluolylen, 2,6-Toluolylen, 1,3-bis-(2-Propylen)-phenylen, Methylen-bisphenylen, mehr bevorzugt 2,4-Toluolylen, 2,6-Toluolylen, insbesondere 2,4-Toluolylen.

q kann vorzugsweise eine ganze Zahl von 1 bis 100 sein, mehr bevorzugt eine ganze Zahl von 10 bis 75, noch mehr bevorzugt eine ganze Zahl von 20 bis 55, insbesondere ein ganze Zahl 25 bis 40.

r kann vorzugsweise eine ganze Zahl von 1 bis 10 sein, mehr bevorzugt eine ganze Zahl von 1 bis 5, noch mehr bevorzugt 1, 2 oder 3, insbesondere 2.

Das Siloxan der Formel (A) wird vorzugsweise erhalten durch die Umsetzung von

- linearem Siloxan (a1) mit der Formel (A1)

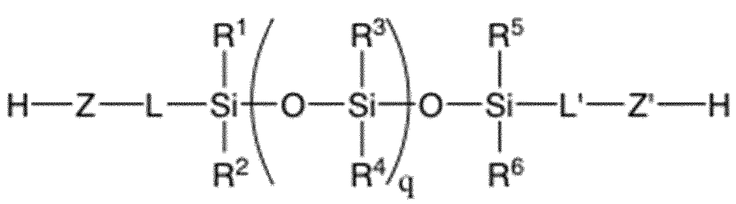

Formel (A1)

wobei $R^1$, $R^2$, $R^3$, $R^4$, $R^5$, R6, Z, Z', L, L' und q wie vorstehend definiert sind,

- Diisocyant (a2)
- Amin (a3) mit der Formel (A3)

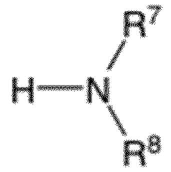

Formel (A3)

wobei $R^7$ und $R^8$ wie vorstehend definiert sind.

In einer bevorzugten Ausführungsform sind bei dem linearen Siloxan mit Formel (A1)

$R^1$, $R^2$, $R^3$, $R^4$, $R^5$, R6 jeweils Methylgruppen,
Z, Z' jeweils Sauerstoff (O),
L, L' jeweils eine Methylengruppe, und
q eine ganze Zahl von 25 bis 40.

Vorzugsweise sind die Diisocyanate (a2) ausgewählt aus 1,6-Hexamethylendiisocyanat (HDI); 1-Isocyanato-3-isocyanatomethyl-3,5,5-trimethylcyclohexan (Isophorondiisocyanat, IPDI); 4,4'-Diisocyanatodicyclohexyl-methan ($H_{12}$MDI); 2,4-Toluoldiisocyanat (2,4-TDI); 2,6-Toluoldisiocynat (2,6-TDI); 2,2'-Diphenylmethandisocyanat (2,2'-MDI), 2,4'-Diphenylmethandisocyanat (2,4'-MDI), 4,4'-Diphenylmethandisocyanat (4,4'-MDI), Naphthalin-1,5-diisocyanat (NDI).

Das Gewichtsverhältnis von Diisocyanat (a2) zu linearem Siloxan (a1) mit der Formel (A1) kann 1 : 1000 bis 4 : 10, bevorzugt 1 : 500 bis 1 : 5, insbesondere 1 : 100 bis 1 : 10 betragen.

Die Amine (a3) sind bevorzugt Dialkylamine, wobei die Alkylgruppen unabhängig 1 bis 6 C-Atome aufweisen, und wobei mindestens eine der Alkylgruppen eine Hydroxylgruppe aufweist. Bevorzugt sind N-Methylethanolamin, Diethanolamin und Bis(2-hydroxypropyl)amin, mehr bevorzugt Diethanolamin und Bis(2-hydroxypropyl)amin, insbesondere Diethanolamin.

Das Gewichtsverhältnis von Amin (a3) zu linearem Siloxan (a1) mit der Formel (A1) kann 1 : 1000 bis 3 : 10, bevorzugt 1 : 500 bis 1 : 10 , insbesondere 1 : 200 bis 1 : 20 betragen.

Die Umsetzung der Komponenten (a1), (a2) und (a3) zum Siloxan mit der Formel (A) kann bevorzugt ohne Lösungsmittel durchgeführt werden.

Alternativ bevorzugt kann die Umsetzung auch in einem organischen Lösungsmittel durchgeführt werden. Geeignete Lösungsmittel sind beispielsweise Ether wie Diethylether, Dioxan oder Tetrahydrofuran, Ester wie Ethyl- oder Butylacetat, Ketone, wie Aceton oder Methylethylketon, aliphatische Kohlenwasserstoffe wie Pentan, Hexan, Heptan, Cyclopentan oder Cyclohexan, halogenierte Kohlenwasserstoffe, wie Methylenchlorid oder Chloroform, Acetonitril, DMF und aromatische Kohlenwasserstoff wie Benzol, Toluol oder Xylol.

Bei Einsatz eines organischen Lösungsmittels kann dieses in Mengen von 10 bis 500 Gew.-%,vorzugsweise 20 bis 400 Gew.-%, insbesondere 35 bis 250 Gew.-% eingesetzt werden, bezogen auf das lineare Siloxan (a1) mit der Formel (A1).

Die oben erwähnte Umsetzung zum Siloxan der Formel (A) kann bevorzugt in der Gegenwart eines Katalysators durchgeführt werden. Der Katalysator ist bevorzugt eine metallorganische Verbindung, wobei das Metall aus Eisen, Kupfer, Zink, Zinn, Bismut und Zirkonium ausgewählt ist. Mehr bevorzugt handelt es sich bei dem Metall um Zinn, Bismut und Zirkonium, insbesondere Bismut. Ganz besonders bevorzugt ist Bi-(III)-neodecanoat.

Der Katalysator kann in einer Menge von 1 bis 1000 Gew.-ppm, bevorzugt von 5 bis 500 Gew.-ppm, insbesondere von 10 bis 200 Gew.-ppm bezogen auf das Gesamtgewicht der Komponenten (a1), (a2) und (a3) eingesetzt werden.

Bevorzugt wird die Umsetzung zur Formel (A) in zwei Schritten durchgeführt:

1) Reaktion des Siloxans (a1) mit der Formel (A1) mit einem Diisocyanat (a2) und
2) Umsetzung der nach 1) erhaltenen Reaktionsmischung mit Amin (a3).

Schritt 1) kann bevorzugt in Gegenwart von Lösungsmittel und/oder Katalysator durchgeführt werden.

Schritt 1) kann bei Temperaturen von 0 bis 100 °C, bevorzugt 10 bis 80 °C, mehr bevorzugt von 30 bis 60 °C durchgeführt werden.

Die Reaktionszeit von Schritt 1) kann 1 bis 120 Minuten, bevorzugt 5 bis 90 Minuten, besonders bevorzugt 15 bis 60 Minuten betragen.

In Schritt 2) wird dem Reaktionsgemisch von Schritt 1) das Amin (a3) zugegeben. Die Temperatur und Reaktionszeit von Schritt 2) entspricht im Wesentlichen der von Schritt 1). Nach Beendigung der Reaktion kann gegebenenfalls das Lösungsmittel entfernt werden, bevorzugt bei einer Temperatur von 20 bis 40 °C und einem (reduzierten) Druck von 1 bis 100 hPa.

In einer bevorzugten Aufführungsform kann die härtbare Mischung, aus welcher der Siloxan-enthaltende Polyurethanschaumstoff erhältlich ist, noch weitere Komponenten enthalten. Beispiele für solche Komponenten sind (iii) Katalysatoren und (iv) Treibmittel.

Als Katalysatoren (iii) können Verbindungen eingesetzt werden, welche die Reaktion der Komponente (i), Polyisocyanat, mit der Komponente (ii), Siloxan, mit der Formel (A) beschleunigen. In Frage kommen beispielsweise tertiäre Amine und/oder organische Metallverbindungen, wie Zink, Zinn, Bismut und Zirkoniumverbindungen, insbesondere organische Zinnverbindungen. Beispielsweise können als Katalysatoren folgende Verbindungen eingesetzt werden: Triethylendiamin, Aminoalkyl- und/oder Aminophenylimidazole und/oder Zinn-(II)-salze von organischen Carbonsäuren. Katalysatoren werden im Allgemeinen in einer Menge von 0,1 bis 5 Gew.-% bezogen auf das Gesamtgewicht der Komponenten (i) und (ii) eingesetzt.

Die Treibmittel (iv) können physikalische und/oder chemische Treibmittel sein. Beispiele für physikalische Treibmittel sind (cyclo)aliphatische Kohlenwasserstoffe, vorzugsweise solche mit 4 bis 8, besonders bevorzugt 4 bis 6 und insbesondere 5 Kohlenstoffatomen, teilhalogenierte Kohlenwasserstoffe oder Ether, Ketone oder Acetate.

Ein chemisches Treibmittel ist ein Treibmittel, das vorzugsweise eine oder mehrere OH-Gruppen enthält. Beispiele für Verbindungen, die eine oder mehrere OH-Gruppen enthalten, sind Wasser (H-OH) und Alkohole mit 1 bis 12 C-Atomen. Besonders bevorzugt ist Wasser.

Es hat sich zur Erreichung der nachstehend beschriebenen physikalischen Eigenschaften gezeigt, dass es vorteilhaft ist, wenn die Wassermenge in der härtbaren Mischung begrenzt ist. Bevorzugt weist die härtbare Mischung einen Wassergehalt von 0,0001 bis 5 Gew.-%, mehr bevorzugt von 0,001 bis 3,0 Gew.-%, noch mehr bevorzugt von 0,01 Gew.-% bis 1,0 Gew.-%, auf. Der Wassergehalt wird hierbei nach der coulometrischen Karl-Fischer-Methode bestimmt. Üblicherweise ergibt sich der Wassergehalt nicht durch Zugabe von Wasser sondern durch den Restwassergehalt der Komponenten der härtbaren Mischung.

Die Umsetzung erfolgt gegebenenfalls in Anwesenheit von (v) Hilfs- und/oder Zusatzstoffen, wie z. B. Füllstoffen, Zellreglern, Zellöffnern, oberflächenaktiven Verbindungen und/oder Stabilisatoren gegen oxidativen, thermischen oder mikrobiellen Abbau oder Alterung. Die Hilfs- und/oder Zusatzstoffe können in einer Menge von 0,1 bis 30 Gew.-%, bevorzugt 0,5 bis 20 Gew.-%, mehr bevorzugt 1 bis 15 Gew.-% vorliegen, bezogen auf Polysiloxan mit der Formel (A).

Um den Siloxan-enthaltenden Polyurethanschaumstoff herzustellen, werden die oben beschriebenen Komponenten in irgendeiner erwünschten Reihenfolge vermengt. Es ist bevorzugt, dass zwei Teilmischung A und B miteinander vermischt werden. Weiterhin ist besonderes bevorzugt, dass die Teilmischung, welche die Komponente (i) enthält, weder die Komponente (ii) noch Treibmittel wie z.B. Wasser enthält. Die Vermischung kann durch übliche Mischgeräte erfolgen, beispielsweise mit einem statischen Mischer oder einem dynamischen Mischer.

Die Umsetzung der Komponenten (i) bis (iv) der härtbaren Zusammensetzung erfolgt bevorzugt bei 20 bis 25 °C, insbesondere bei 23 °C.

Die vorstehend erläuterten Komponenten (i) bis (iv) werden bevorzugt so gewählt, dass ein offenzelliger Schaumstoff auf Basis eines (quervernetzten), Siloxan-enthaltenden Polyurethans, insbesondere ein Schaumstoff mit den nachfolgend beschriebenen physikalischen Eigenschaften, erhalten wird.

Es hat sich gezeigt, dass vorstehend genannte Aufgaben unerwartet vorteilhaft gelöst werden können, wenn der Siloxan-enthaltende Polyurethanschaumstoff eine spezielle Zugfestigkeit, eine spezielle Bruchdehnung und eine spezielle Härte aufweist. In einer bevorzugten Ausführungsform weist der Siloxan-enthaltende Polyurethanschaumstoff eine Zugfestigkeit von 100 kPa bis 100 MPa, mehr bevorzugt 500 kPa bis 50 MPa, noch mehr bevorzugt 800 kPa bis 10 MPa, gemessen gemäß DIN 53571, auf. Ferner weist der Siloxan-enthaltende Polyurethanschaumstoff bevorzugt eine Bruchdehnung von 100 % bis 350 %, mehr bevorzugt von 160 % bis 320 %, noch mehr bevorzugt von 180 % bis 300 %, gemessen gemäß DIN 53571, auf. Zudem weist der Siloxan-enthaltende Polyurethanschaumstoff bevorzugt eine Härte von 20 bis 70 Shore A, mehr bevorzugt von 30 bis 60 Shore A, noch mehr bevorzugt von 40 bis 50 Shore A, gemessen gemäß DIN 53505, auf.

Es hat sich ferner gezeigt, dass vorstehend genannte Aufgaben unerwartet vorteilhaft gelöst werden können, wenn der Siloxan-enthaltende Polyurethanschaumstoff eine spezielle Luftdurchlässigkeit aufweist. In einer bevorzugten Ausführungsform weist der Siloxan-enthaltende Schaumstoff eine Luftdurchlässigkeit von 1000 bis 8000 l/($m^2$sec), mehr bevorzugt von 2000 bis 7500 l/($m^2$sec), noch mehr bevorzugt von 2500 bis 7000 l/($m^2$sec), insbesondere von 3000 bis 6500 l/($m^2$sec), gemessen gemäß DIN EN ISO 9237, auf.

Es hat sich weiterhin gezeigt, dass vorstehend genannte Aufgaben unerwartet vorteilhaft gelöst werden können, wenn der Siloxan-enthaltende Polyurethanschaumstoff viskoelastisches Verhalten zeigt. Hierunter wird verstanden,

dass das Verhalten des Siloxan-enthaltende Polyurethanschaumstoffs auf Beanspruchung so aussieht, wie die Kombination aus einem elastischen Feststoff und einer viskosen Flüssigkeit. Das viskoelastische Verhalten kann durch einen Torsionsschwingungsversuch gemäß DIN 53445 charakterisiert werden. Es ist bevorzugt, dass der Schaumstoff bei der Bestimmung gemäß DIN 53445 bei 23 °C einen mechanischen Verlustfaktor von 0,1 bis 1,0, mehr bevorzugt von 0,15 bis 0,8, noch mehr bevorzugt von 0,2 bis 0,6 aufweist.

Es hat sich weiterhin gezeigt, dass vorstehend genannte Aufgaben unerwartet vorteilhaft gelöst werden können, wenn der Siloxan-enthaltende Polyurethanschaumstoff eine Rohdichte zwischen 10 und 120 $kg/m^3$, mehr bevorzugt zwischen 12 und 50 $kg/m^3$, insbesondere zwischen 15 und 45 $kg/m^3$ aufweist, gemessen gemäß DIN EN ISO 845 (2009). Als besonders vorteilhaft hat sich ein Siloxan-enthaltender Polyurethanschaumstoff mit einer Rohdichte im Bereich zwischen 24 und 28 $kg/m^3$ erwiesen.

Es hat sich weiterhin gezeigt, dass vorstehend genannte Aufgaben unerwartet vorteilhaft gelöst werden können, wenn der Siloxan-enthaltende Polyurethanschaumstoff Substanzen mit antimikrobieller Wirkung (v) enthält.

Eine mögliche Substanz mit antimikrobieller Wirkung ist Silber und kann in Form von Silberionen oder in Form von atomarem Silber in dem Siloxan-enthaltenden Polyurethanschaumstoff enthalten sein. Bevorzugt wird nach der Herstellung des Siloxan-enthaltenden Polyurethanschaumstoffs eine Silberbeschichtung aufgebracht. Alternativ kann das Silber bereits in die härtbare Zusammensetzung miteingebracht werden. Bevorzugt enthält der Siloxan-enthaltenden Polyurethanschaumstoff 0,000001 bis 0,1 Gew.-%, mehr bevorzugt 0,0001 bis 0,01 Gew.-% Silber, bezogen auf das Gesamtgewicht des Siloxan-enthaltenden Polyurethanschaumstoffs.

Des Weiteren kann der Siloxan-enthaltende Polyurethanschaumstoff auch organische Substanzen mit antimikrobieller Wirkung enthalten. Bei der Substanz mit antimikrobieller Wirkung kann es sich beispielsweise um Substanzen mit Amino- oder Iminogruppen handeln. Weiterhin kann es sich bei der Substanz mit antimikrobieller Wirkung um antimikrobiell wirksame Metallkationen handeln, insbesondere um Silberkationen, beispielsweise um einen Komplex von 1-Vinyl-2-Pyrrolidone mit Silberkationen. Besonders geeignete Substanzen mit antimikrobieller Wirkung sind weiterhin Biguanid-Derivate, wie Chlorhexidin, oder Polybiguanide, wie Polyethylenbiguanid (PEB), Polytetramethylenbiguanid (PTMB) oder Polyethylenhexamethylenbiguanid (PEHMB). Ein besonders bevorzugtes Polybiguanid ist Polyhexamethylenbiguanid (PHMB bzw. Polyhexanid). Weitere geeignete Substanzen mit antimikrobieller Wirkung sind Polyguanidine, wie zum Beispiel Polyhexamethylenguanidine (PHMG), *N*-Octyl-1-[10-(4-Octyliminopyridin-1-yl)decyl]pyridin-4-imin (Octenidin), quartäre Ammoniumverbindungen, wie zum Beispiel Benzalkoniumchlorid oder Cetylpyridiniumchlorid, Triazine, wie zum Beispiel 1-(3-Chloroallyl)-3,5,7-triaza-1-azonia-adamantan-Chlorid oder die Ammoniumverbindung Taurolidin.

Bevorzugt wird der Siloxan-enthaltende Polyurethanschaumstoff mit den vorstehend erläuterten Substanzen mit antimikrobieller Wirkung imprägniert oder beschichtet.

Substanzen mit antimikrobieller Wirkung sind üblicherweise in dem Polyurethanschaumstoff in einer Menge von 0 bis 30 Gew.-%, bevorzugt von 0,1 bis 15 Gew.-%, bezogen auf das Gesamtgewicht des Siloxan-enthaltenden Polyurethanschaumstoffs, enthalten.

In einer bevorzugten Ausführungsform wird der Siloxan-enthaltende Polyurethanschaumstoff in trockenem Zustand eingesetzt. Bevorzugt ist somit der Schaumstoff z.B. nicht mit einer Aktivierungslösung (wie z.B. Ringerlösung) getränkt.

In einer alternativ bevorzugten Ausführungsform umfasst die Wundkontaktschicht (a) einen Siloxan-funktionalisierten Polyurethanschaumstoff. Ein Siloxan-funktionalisierter Polyurethanschaumstoff kann als ein Polyurethanschaumstoff betrachtet werden, bei dem nach der Aufschäumung noch vorhandene reaktive Gruppen wie Hydroxy- oder Aminogruppen mit einer reaktiven Siloxankomponente umgesetzt wurden, d.h. eine reaktive Gruppe des Polyurethanschaumes wurde als Siloxanrest funktionalisiert. Hierbei ist dann der Siloxanrest mithilfe einer kovalenten Bindung an den Polyurethanschaum gebunden.

In einer bevorzugten Ausführungsform besteht die den Siloxan-enthaltenden Polyurethanschaumstoff oder einen Siloxan-funktionalisierten Polyurethanschaumstoff enthaltende Wundkontaktschicht (a) aus Siloxan-enthaltendem Polyurethanschaumstoff oder einem Siloxan-funktionalisierten Polyurethanschaumstoff. Alle vorstehend aufgeführten Erläuterungen zu bevorzugten Ausführungsformen zu dem Siloxan-enthaltenden Polyurethanschaumstoff und dem Siloxan-funktionalisierten Polyurethanschaumstoff finden sowohl einzeln als auch in Kombination auch für die erfindungsgemäße Verwendung Anwendung.

In einer bevorzugten Ausführungsform der Erfindung weist die Wundkontaktschicht (a) eine Schichtdicke von 1 bis 15 mm, bevorzugt von 2 bis 12 mm, insbesondere von 3 bis 10 mm auf.

In einer bevorzugten Ausführungsform der Erfindung ist die Wundkontaktschicht (a) dünner als die einen hydrophilen Schaumstoff umfassende Schicht (b).

In einer bevorzugten Ausführungsform der Erfindung weist die Wundkontaktschicht (a) Öffnungen bzw. Perforationen auf. Diese Öffnungen bzw. Perforationen können regelmäßige oder unregelmäßige Geometrien aufweisen. Vorzugsweise weisen die Öffnungen bzw. Perforationen einen effektiven Durchmesser von 0.5 bis 15 mm, mehr bevorzugt 2.0 bis 10 mm, auf. Es wird vermutet, dass hierdurch der Transport von Debris und/oder Flüssigkeit in die hydrophile

Schicht (b) zu unterstützt wird.

Die Wundkontaktschicht (a) ist vorzugweise so angeordnet, dass sie wundseitig die hydrophile Schicht (b) flächig bedeckt. Im Rahmen der vorliegenden Erfindung bedeutet eine flächige Anordnung zweier Schichten, dass diese zwei Schichten zu mindestens 75%, bevorzugt zu mindestens 85%, insbesondere zu 100% ihrer Flächen übereinander angeordnet sind. Somit bedeutet die flächige Bedeckung der hydrophilen Schicht (b) mit der Wundschicht (a), dass die Wundschicht (a) mindestens 75%, bevorzugt mindestens 85%, insbesondere 100% der hydrophilen Schicht (b) bedeckt.

Die hydrophile Schicht (b) umfasst einen hydrophilen Schaumstoff Als hydrophile Schaumstoffe werden in Rahmen der vorliegenden Erfindung Schaumstoffe betrachtet, welche einen Wasseranteil von mindestens 10% umfassen können. Beispiele sind dem Fachmann bekannt, zum Beispiel hydrophiler Polyurethanschaumstoff wie Polyurethan-Ester, Polyurethan-Ether, Polyurethan-Polyharnstoff-Copolymer, Polylactide, Chitosan, Kollagen oder hydrophiler Polyvinylalkoholschaumstoff.

In einer bevorzugten Ausführungsform besteht die hydrophile Schicht aus einem hydrophilen Schaumstoff.

In einer bevorzugten Ausführungsform wird der von der hydrophilen Schicht (b) umfasste hydrophile Schaumstoff mit Substanzen mit antimikrobieller Wirkung imprägniert oder beschichtet.

Alle vorstehend aufgeführten Erläuterungen zu bevorzugten Ausführungsformen zu den Substanzen mit antimikrobieller Wirkung finden sowohl einzeln als auch in Kombination auch für die erfindungsgemäße Verwendung Anwendung.

Weiterhin kann die hydrophile Schicht vorzugsweise mit einer pharmazeutisch verträglichen Lösung aktiviert sein. Pharmazeutisch verträgliche Lösungen sind dem Fachmann bekannt. Es ist besonders bevorzugt, dass der hydrophile Schaumstoff mit einer Ringerlösung aktiviert wird.

In einer bevorzugten Ausführungsform weist der von der hydrophilen Schicht (b) umfasste hydrophile Schaumstoff ein Absorptionsvermögen von Kochsalzlösung zwischen 2 g/g und 30 g/g), bevorzugt zwischen 8 g/g und 12 g/g auf, gemessen nach DIN EN 13726-1.

In einer bevorzugten Ausführungsform umfasst die hydrophile Schicht (b) einen hydrophilen Polyurethan- oder Polyvinylalkoholschaumstoff.

In einer bevorzugten Ausführungsform umfasst die hydrophile Schicht (b) einen hydrophilen Polyvinylalkoholschaumstoff.

In einer bevorzugten Ausführungsform umfasst die hydrophile Schicht (b) einen hydrophilen Polyurethanschaumstoff.

Insbesondere umfasst die hydrophile Schicht (b) einer erfindungsgemäßen Wundauflage einen hydrophilen Polyurethanschaum, der einen Wasseranteil von mindestens 10 Gew.-% Wasser, bevorzugt mindestens 20 Gew.-%, insbesondere mindestens 30 Gew.-% und ganz besonders bevorzugt mindestens 35 Gew.-% Wasser umfasst. Hierbei ist weiterhin bevorzugt vorgesehen, dass der Polyurethanschaum einen Wasseranteil von höchstens 80 Gew.-% Wasser, insbesondere höchstens 70 Gew.-% und ganz besonders bevorzugt von höchstens 65 Gew.-% Wasser umfasst. Insbesondere ist dieser Wasseranteil homogen in der Polyurethanmatrix des Schaums verteilt. Insbesondere umfasst der hydrophile Polyurethanschaum dabei einen Wasseranteil von mindestens 10 Gew.-% und höchstens 80 Gew.-% Wasser, wobei das Wasser insbesondere homogen in dem Polyurethanschaum verteilt ist.

Im Zusammenhang mit der vorliegenden Erfindung soll- falls nichts anderes angegeben ist - jeder Gehalt eines Inhaltstoffes in Gewichtsprozent (Gew.-%) bezogen auf das Gewicht der den Inhaltsstoff umfassenden Komponente verstanden sein. Zur Überprüfung der Menge an Wasser in der jeweiligen Komponente soll im Zusammenhang mit der vorliegenden Erfindung die Norm DIN EN 14079 herangezogen werden, wobei die Menge an Wasser wie folgt berechnet wird:

$$W_w = \frac{W_g - W_t}{W_g} \cdot 100\%$$

mit

$W_w$ = Gewicht des Wassers in % bezogen auf das Gesamtgewicht der Komponente
$W_g$ = Gewicht der Wasser enthaltenden Komponente
Wt = Gewicht der trockenen Komponente.

Damit soll im Zusammenhang mit der vorliegenden Erfindung unter einem hydrophilen Polyurethanschaum mit einem Wasseranteil von mindestens 10 Gew.-% ein solcher hydrophiler Polyurethanschaum verstanden sein, der mindestens 10 Gew.-% Wasser umfassen kann, wobei das Wasser von dem hydrophilen Polyurethanschaum freigesetzt werden kann. Im Unterschied hierzu ist nicht der Anteil an Wasser zu verstehen, der möglicherweise zur Bildung bei der Polymerisation der Ausgangsprodukte des hydrophilen Polyurethanschaums verwendet wird. Dieses Wasser wird

kovalent gebunden und steht nicht der Wundbehandlung zur Verfügung. Darüber hinaus soll auch nicht ein Wasseranteil verstanden sein, der produktionsbedingt bei der Herstellung des Schaums verwendet wird. Dieser Wasseranteil wird nach oder während der Bildung des hydrophilen Polyurethanschaums dem Schaum meist durch Trocknen, beispielsweise durch Trocknen in einem Ofen, entzogen und steht somit auch nicht der Wundbehandlung zur Verfügung. Damit weist eine erfindungsgemäße Wundauflage einen hydrophilen Polyurethanschaum auf, der einen Wasseranteil umfassen kann, der deutlich einen eventuell durch die Herstellung nach Trocknung bedingten Restgehalt an Wasser übersteigt.

Der von der hydrophilen Schicht umfasste hydrophile Polyurethanschaum weist vorzugsweise einen Retentionswert R von mindestens 20 % auf. Hierbei ist weiterhin bevorzugt vorgesehen, dass der hydrophile Polyurethanschaum einen Retentionswert R von mindestens 30 %, insbesondere von mindestens 40 % und ganz besonders bevorzugt von mindestens 50 % aufweist. Unabhängig hiervon kann weiterhin bevorzugt vorgesehen sein, dass die Wundauflage einen hydrophilen Polyurethanschaum aufweist, der einen Retentionswert R von höchstens 90 %, insbesondere von höchstens 80 % und ganz besonders von höchstens 70 % aufweist. Der Retentionswert R wird dabei gemäß der nachfolgend beschriebenen Methode bestimmt. Ganz besonders bevorzugt umfasst die hydrophile Schicht (b) einen hydrophilen Polyurethanschaum, der einen Wasseranteil von mindestens 10 Gew.-% umfasst, wobei der Wasseranteil dem Retentionswert R des Polyurethanschaums entspricht. Der Retentionswert R wird dabei gemäß der nachfolgend beschriebenen Methode bestimmt.

Der Retentionswert R beschreibt die Menge Wasser, die der Polyurethanschaum in seine Polyurethanmatrix maximal einbinden kann, wobei das Wasser, das in die Poren aufgenommen werden könnte, unberücksichtigt bleibt. Der Retentionswert wird bestimmt, indem ein Probenstück von 5 cm × 5 cm (unter Normklima gelagert) aus einem hydrophilen Polyurethanschaum mit einer Dicke von höchstens 5 mm ausgestanzt und dessen Gewicht unter Normklimabedingungen vermessen wird. Das Probenstück wird hiernach einer freien Absorption mit Wasser analog DIN EN 13726-1 unterworfen. Das Wasser, das von den Poren aufgenommen wurde, wird aus dem Probenstück mittels einer Rolle (Gewicht 5000 g, Durchmesser 10 cm, Breite 5 cm) herausgequetscht, in dem die Probe mehrmals zwischen frische Zellstofftücher gelegt und mit der Rolle überrollt wird. Dieser Vorgang wird solange wiederholt, bis keine Wasserabsorption in den Zellstofftüchern erkennbar ist. Zur Bestimmung des Retentionswertes R wird der Wasseranteil $W_{ww}$, der nach dem Absorbieren und dem Ausquetschen in dem Polyurethanschaum enthalten ist, gemäß DIN EN 14079 gemessen und wie folgt berechnet.:

$$R = W_{ww} = \frac{W_{gg} - W_{tt}}{W_{gg}}$$

wobei gilt:

- $W_{ww}$ = das Gewicht des Wassers, das nach der Absorption und dem Ausquetschen in dem Polyurethanschaum enthalten ist,
- $W_{tt}$ = das Gewicht des Probenstücks nach dem Trocknen ist und
- $W_{gg}$= das Gewicht des Probenstücks nach Absorption und nach Ausquetschen ist.

Somit kann eine Wundauflage bereitgestellt werden, die bestens für die moderne Wundbehandlung konditioniert ist, da die Wundauflage durch den Wassergehalt in der hydrophilen Schicht (b), insbesondere in der einen hydrophilen Polyurethanschaum umfassenden hydrophilen Schicht, einer zu behandelnden Wundoberfläche sofort nach der Applikation ein besonders wundheilungsförderndes Klima zur Verfügung stellt. Die Wunde wird von Anfang an mit Feuchtigkeit oder Wasser versorgt, wobei die Wundauflage gleichzeitig durch den absorbierenden hydrophilen Schaumstoff eine ausreichende Absorptionskapazität aufweist. Hierdurch werden die Wundheilung eventuell negativ beeinflussende Faktoren der Wundoberfläche entzogen und gleichzeitig Feuchtigkeit und Wasser in einer ausreichenden Menge zur Verfügung gestellt. Diese Wundauflage ist hervorragend dazu geeignet, in der Epithelisierungs- oder Granulationsphase der Wundheilung eingesetzt zu werden.

Damit kann die erfindungsgemäße Wundauflage in natürlicher Weise die Granulation und/oder die Epithelisierung der Wunde in besonderem Maße fördern.

In einer bevorzugten Ausführungsform weist der von der hydrophilen Schicht (b) umfasste hydrophile Polyurethanschaum ein Quellvermögen $\Delta V_0$ von höchstens 80 % auf. Insbesondere bevorzugt weist der hydrophile Polyurethanschaumstoff ein Quellvermögen $\Delta V_0$ von höchstens 60 %, insbesondere von höchstens 40 %, insbesondere von höchstens 30 % und ganz besonders bevorzugt von höchstens 20 % auf. Dabei kann weiterhin vorteilhaft vorgesehen sein, dass der hydrophile Polyurethanschaumstoff ein Restquellvermögen $\Delta V_0$ von mindestens 5 % aufweist.

Hierbei soll unter dem Quellvermögen $\Delta V_0$ eines hydrophilen Polyurethanschaumstoffs die Volumenzunahme verstanden sein, die ein hydrophiler Polyurethanschaumstoff, der seine Absorptionskapazität vollständig erschöpft hat, im Vergleich zu einem Polymerschaumstoff mit einem Wassergehalt von 10 Gew.-% Wasser erfährt. Das Quellvermögen

kann dabei gemäß dem nachfolgend beschriebenen Test ermittelt werden.

Das Quellvermögen $\Delta V_0$ eines Polyurethanschaums beschreibt die Volumenänderung, die ein Polyurethanschaumstoff mit einem Wassergehalt von 10 Gew.-% Wasser erfährt, nachdem er seine maximale Absorption erreicht hat. Zur Bestimmung des Quellvermögens $\Delta V_0$ werden die räumlichen Abmessungen eines Probenstücks des Polymerschaumstoffs mit einem Wassergehalt von 10 Gew.-% Wasser und die räumlichen Abmessungen dieses Probenstücks nach vollständiger Absorption gemäß der freien Absorption nach DIN EN 13726-1 bestimmt. Zur Bestimmung der Dicke (Höhe) wird ein Dickenmessgerät mit 25 $cm^2$ Teller verwendet, wobei eine Belastung von 2 $g/cm^2$ gemäß EN ISO 9073-2 eingestellt wird. Die laterale Ausdehnung (Länge, Breite) wird mittels einer Schieblehre bestimmt, ohne dass das Probenstück deformiert wird. Zur Bestimmung der Ausdehnung wird das jeweilige Probenstück spannungsfrei auf eine glatte Oberfläche abgelegt. Daraus lässt sich das entsprechende Volumen auf bekannte Weise berechnen (V = l·b·h). Die Volumenänderung nach Absorption entspricht dem Quellvermögen $\Delta V_0$ des Polyurethanschaumstoffs mit einem Wassergehalt von 10 Gew.-% Wasser, wobei alle drei Raumrichtungen beachtet werden. Das Quellvermögen $\Delta V_0$ kann wie folgt berechnet werden.

$$\Delta V_0 = \frac{V_1 - V_0}{V_0}$$

wobei gilt

$V_0$ ist das das Volumen des Probenstückstücks mit einem Wassergehalt von 10 Gew.-% Wasser, und
$V_1$ ist das Volumen des Probenstückstücks nach vollständiger Absorption.

Als hydrophiler Polyurethanschaumstoff kann im Zusammenhang mit der vorliegenden Erfindung jeder heute in der modernen Wundbehandlung übliche hydrophile Polyurethanschaumstoff mit einem Wasseranteil von mindestens 10 % verwendet werden, der eine ausreichende Absorption aufweist. Dabei ist im Zusammenhang mit der vorliegenden Erfindung unter einem hydrophilen Polyurethanschaumstoff ein solcher Polyurethanschaum zu verstehen, der eine Flüssigkeit in seine Polyurethanmatrix und in seine Poren aufnehmen und speichern, somit absorbieren kann, und zumindest einen Teil der aufgenommenen Flüssigkeit wieder abgeben kann. Als hydrophile Polymerschaumstoffe sind hierbei insbesondere offenporige, hydrophile Polyurethanschaumstoffe geeignet. Demgemäß umfasst eine besonders bevorzugte Wundauflage eine hydrophile Schicht, die einen offenporigen, hydrophilen Polyurethanschaumstoff umfasst.

Erfindungsgemäß sollen solche hydrophile Polyurethanschaumstoffe eingesetzt werden, die eine hohe Absorptionskapazität aufweisen. Diese Absorptionskapazität soll vorhanden sein, selbst wenn der Polyurethanschaumstoff einen Anteil seines Eigengewichtes an Wasser in seine Polymermatrix aufgenommen hat. Gemäß einer Weiterbildung der Erfindung umfasst damit die hydrophile Schicht (b) einen Polyurethanschaumstoff, der eine freie Absorption A2 von mindestens 10 g/g bis 30 g/g, insbesondere mindestens 12 g/g bis 30 g/g und ganz besonders bevorzugt von mindestens 15 g/g bis 30 g/g aufweist, wobei die freie Absorption A2 gemäß der DIN-EN 13726-1 (2002) bestimmt wird. Hierbei ist die freie Absorption A2 die freie Absorption des Wasser-enthaltenden Polyurethanschaumstoffs.

Der von der hydrophilen Schicht (b) umfasste hydrophilen Polyurethanschaumstoff weist eine durchschnittliche Porengröße von weniger als 1000 $\mu$m, insbesondere 100 bis 1000 $\mu$m, insbesondere 100 bis 500 $\mu$m und ganz besonders bevorzugt 100 bis 300 $\mu$m auf. Weiterhin bevorzugte hydrophile Polyurethanschaumstoffe weisen eine Dichte von weniger als 150 $kg/m^3$, insbesondere weniger als 140 $kg/m^3$ und ganz besonders bevorzugt 12 bis 120 $kg/m^3$ auf.

Als besonders vorteilhafte Ausführungen haben sich weiterhin Wundauflagen gezeigt, deren hydrophile Schicht (b) einen Polyurethanschaum umfasst, dessen Schichtdicke 1 mm bis 15 mm, insbesondere 2 mm bis 10 mm aufweist.

In einer bevorzugten Ausführungsform umfasst die vorliegende Wundauflage weiter

(c) eine Trägerschicht und/oder

(d) eine Schicht umfassend den Siloxan-enthaltenden Polyurethanschaumstoff oder einen Siloxan-funktionalisierten Polyurethanschaum, welche auf der wundabgewandten Seite der hydrophilen Schicht (b) umfassend einen hydrophilen Schaumstoff angeordnet ist.

Diese Trägerschicht (c) kann aus verschiedenen Materialien bestehen, beispielsweise aus Polymerfilmen oder Polymerschaumstoffen. Diese Trägerschicht (c) kann direkten Kontakt oder indirekten Kontakt zu der wundabgewandten Seite der hydrophilen Schicht (b) aufweisen, d.h. die Trägerschicht (c) kann bevorzugt auf der wundabgewandten Seite der hydrophilen Schicht (b) angeordnet sein. Bei einem direkten Kontakt wird die Trägerschicht (c) direkt auf die hydrophile Schicht auflaminiert, wogegen bei einem indirekten Kontakt die Trägerschicht (c) mittels eines Adhäsivs auf die hydrophile Schicht (b) aufgebracht ist. Dabei kann das Adhäsiv flächig oder lediglich in Teilbereichen zwischen der Trägerschicht

(c) und hydrophilen Schicht (b) aufgebracht sein.

Als Trägerschicht (c) einer erfindungsgemäßen Wundauflage können insbesondere Polymerfilme oder Polymerschaumstoffe eingesetzt werden. Ganz besonders bevorzugt sind Polymerfilme oder Polymerschäume, die wasserundurchlässig sind und die eine hohe Wasserdampfdurchlässigkeit aufweisen. Hierzu sind besonders Filme oder Schäume geeignet, die aus Polyurethan, Polyetherurethan, Polyesterurethan, Polyether-Polyamid-Copolymeren, Polyacrylat oder Polymethacrylat gefertigt werden. Insbesondere ist als Trägerschicht (c) ein wasserundurchlässiger und wasserdampfdurchlässiger Polyurethanfilm oder ein wasserundurchlässiger und wasserdampfdurchlässiger Polyurethanschaum geeignet. Insbesondere ist als Polymerfilm ein Polyurethanfilm, Polyesterurethanfilm oder Polyetherurethanfilm bevorzugt. Ganz besonders sind aber auch solche Polymerfilme bevorzugt, die eine Dicke von 15 bis 50 $\mu$m, insbesondere 20 bis 40 $\mu$m und ganz besonders bevorzugt von 25 bis 30 $\mu$m aufweisen. Die Wasserdampfdurchlässigkeit des Polymerfilms der Wundauflage weist vorzugsweise mindestens 750 g/m$^2$/24 Std., insbesondere mindestens 1000 g/m$^2$/24 Std. und ganz besonders bevorzugt mindestens 2000 g/m$^2$/24 Std. auf (gemessen nach DIN EN 13726). In besonders bevorzugten Ausführungsformen weisen diese Filme einen feuchtigkeitsdichten, klebenden Randabschnitt auf. Dieser Randabschnitt gewährleistet, dass die Wundauflage an seinem bestimmungsgemäßen Ort appliziert und fixiert werden kann. Darüber hinaus ist sichergestellt, dass keine Flüssigkeit zwischen der Folie und der die zu behandelnde Fläche umgebenden Haut austreten kann. Als besonders bevorzugt sind solche Klebstoffe zu betrachten, die in einem dünnen Auftrag von 20 bis 35 g/m$^2$ zusammen mit dem Film eine Wasserdampfdurchlässigkeit von mindestens 800 g/m$^2$/24 Std. und vorzugsweise von mindestens 1000 g/m$^2$/24 Std. (gemessen nach DIN EN 13726) aufweisen. In einer bevorzugten Ausführungsform wird eine Trägerschicht (c) verwendet, wenn die hydrophile Schicht (b) einen hydrophilen Polyurethanschaumstoff umfasst. Durch die Trägerschicht (c) kann die vorliegende Wundauflage stabilisiert werden, was die Anwendungssicherheit beim Entfernen der Wundauflage erhöht.

Im Falle, dass die hydrophile Schicht (b) einen Polyvinylalkoholschaumstoff umfasst, kann vorzugsweise auf eine Trägerschicht (c) verzichtet werden.

In einer bevorzugten Ausführungsform kann die Trägerschicht (c) die Abdeckschicht sein, d.h. die Schicht, mit welcher die Wunde auf der wundabgewandten Seite abgedeckt wird. In diesem Fall sollte die Größe der Abdeckschicht so bemessen sein, dass die Abdeckschicht im Bereich der Wundumgebung befestigt werden kann.

Die Schicht (d) umfasst den Siloxan-enthaltenden Polyurethanschaumstoff oder einen Siloxan-funktionalisierten Polyurethanschaum, welcher auf der wundabgewandten Seite der einen hydrophilen Schaumstoff umfassenden hydrophilen Schicht (b) angeordnet sein kann. Für den Aufbau bzw. die Zusammensetzung der Schicht (d) gilt das Gleiche wie bei der oben beschriebenen Wundkontaktschicht (a). Folglich ist die entsprechende Wundauflage symmetrisch aufgebaut und weist eine "Sandwichstruktur" auf. Bei einer Wundauflage mit der symmetrischen Sandwichstruktur ist die Anwendungssicherheit durch beidseitige Verwendbarkeit der Wundauflage signifikant erhöht. Weiterhin kann so eine Wundauflage in Wundkavitäten, bei denen Kontakt zu den gegenüberliegenden Oberflächen der Wundauflage mit dem Wundgrund zu erwarten sind, angewendet werden.

Gemäß einem weiterführenden Gedanken ist auch ein Verfahren zur Herstellung der vorliegenden Wundauflage Gegenstand der vorliegenden Erfindung. Dieses Verfahren umfasst das Verbinden der Wundkontaktschicht (a) mit der hydrophilen Schicht (b). Vorzugsweise umfasst das Verbinden der Schichten das Verkleben mit einem Adhäsiv, Ultraschallschweißen in einem kontinuierlichen Laminierprozess oder einem Thermobondingprozess.

Das Verkleben mit einem Adhäsiv kann mit den dem Fachmann bekannten Adhäsiven durchgeführt werden. In einer bevorzugten Ausführungsform wird das Adhäsiv so aufgetragen, dass die Schichten flächig fest miteinander verbunden werden. Beim Ultraschallschweißen in einem kontinuierlichen Laminierprozess wird die erfindungsgemäße Wundauflage als Verbund aus den Schichten durch Verschweißung der einzelnen aufeinander liegenden Schichten hergestellt. In einer bevorzugten Ausführungsform wird diese Verschweißung durch eine hochfrequente mechanische Schwingung im Bereich von 20 bis 35 kHz erreicht. Diese Frequenz führt zur Erwärmung durch Molekular- und Grenzflächenreibung, was zur Verzahnung und/oder Verhakung der betroffenen Schichten führt.

Bei einem Thermobonding-Prozess wird das Verbinden der Wundkontaktschicht (a) mit der hydrophilen Schicht (b) durch die Zufuhr von Energie in Form von Wärme erreicht, beispielsweise durch Flammkaschierung. Bei der Flammkaschierung wird das Schaummaterial an der Oberfläche angeschmolzen mit dem Ziel, diese Schmelze als Kleber zu nutzen. So erreicht man eine homogene Verbindung der Klebepartner. Ein weiterer Aspekt der vorliegenden Erfindung ist eine pharmazeutisch verträgliche Lösung, insbesondere Ringerlösung, zur Verwendung in der Behandlung von Wunden, insbesondere in der Granulationsphase, wobei die pharmazeutisch verträgliche Lösung in einer hydrophilen Schicht einer Wundauflage enthalten ist, wobei die Wundauflage umfasst:

(a) Wundkontaktschicht umfassend den Siloxan-enthaltenden Polyurethanschaumstoff oder ein Siloxan-funktionalisiertes Polyurethan,
(b) hydrophile Schicht umfassend einen hydrophilen Schaumstoff.

Alle vorstehend aufgeführten Erläuterungen zu bevorzugten Ausführungsformen bezüglich der pharmazeutisch

verträglichen Lösung, insbesondere Ringerlösung, der Wundkontaktschicht (a) und der hydrophile Schicht (b) finden sowohl einzeln als auch in Kombination für die erfindungsgemäße Verwendung Anwendung.

Im Sinne dieser Erfindung liegt üblicherweise eine Wunde vor, wenn an einer äußeren oder inneren Körperoberfläche der Gewebezusammenhang getrennt wurde.

Es gibt unterschiedliche Arten von Wunden. So wird unter einer primär heilenden Wunde eine Wunde mit nicht klaffenden Wundrändern verstanden, welche sich durch komplikationslose Heilung ohne Infektion auszeichnet. Diese Wunden kommen häufig in gut durchbluteten Körperteilen vor. Die nicht klaffenden und damit eng aneinander liegenden Wundränder können beispielsweise durch einen (chirurgischen) Schnitt verursacht worden sein. Erfolgt keine weitere Behandlung, schließt sich die Wunde komplikationslos.

Weitere Wunden sind die sekundär heilenden Wunden. Unter einer sekundär heilenden Wunde wird eine Wunde verstanden, wenn a) ein Verlust des Gewebes vorliegt und/oder b) eine Verkeimung eingetreten ist, welche die primäre Heilung verhindert. Den Verlust an Gewebe kann der Organismus durch neu zu bildendes Gewebe und Überhäutung ausgleichen. Dies führt im Rahmen der sekundären Wundheilung über die Bildung eines Granulationsgewebes bis zum narbigen Ersatz der Gewebslücke.

In einer bevorzugten Ausführungsform der vorliegenden Erfindung ist die sekundär heilende Wunde eine mechanische Wunde, eine thermische Wunde, eine durch chemische Substanzen oder durch Strahlung verursachte Wunde.

Durch äußere Gewalteinwirkung können mechanische Wunden entstehen. Hierzu zählen beispielsweise Schnitt-, Stich-, Platz-, Quetsch-, Schürf-, Kratz-, Biss- und Schusswunden.

Thermische Wunden werden im Wesentlichen durch starke Wärme oder Kälteeinwirkung verursacht. Hierzu zählen beispielsweise Verbrennungen, Verbrühungen, Erfrierungen sowie durch elektrischen Strom verursachte Verletzungen.

Unter chemischen Wunden werden Verätzungen verstanden. Diese können beispielsweise durch saure, alkalische, oxidierende und/oder reduzierende Substanzen verursacht werden.

Durch Strahlung verursachte Wunden werden auch als aktinische Wunden bezeichnet. Diese werden beispielsweise durch ionisierende Strahlung ausgelöst und können ein ähnliches Erscheinungsbild wie Verbrennungswunden aufweisen.

Die inflammatorische Phase tritt üblicherweise direkt nach dem Trauma auf und dauert in etwa drei Tage. Sie ist gekennzeichnet durch Gefäßkontraktion, Aktivierung der Gerinnungskaskade und komplexe immunologische Abläufe. Es kommt in der Regel zur Ausbildung eines Fibrinnetzes, welches die Wunde verschließt und nach außen schützt. Durch die Freisetzung vasoaktiver Substanzen (z.B. Histamin und Serotonin) kann eine lokale Entzündungsreaktion hervorgerufen werden. Die umliegenden Gefäße können sich erweitern und durch eine gesteigerte Kapillarpermeabilität können Leukozyten zum Entzündungsort wandern. Diese können Mikroorganismen und Gewebsnekrosen beseitigen. Dadurch kann eine Reinigung der Wunde erfolgen.

Die darauffolgende Proliferations- oder Granulationsphase beginnt üblicherweise etwa am zweiten Tag nach der Wundentstehung und kann z.B. bis zu 14 Tage andauern. Es erfolgt der Aufbau von neuem Gewebe mit Gefäßeinsprossung und Defektauffüllung durch Granulationsgewebe. Dies ist die Grundvoraussetzung für die spätere Epithelisierung. Fibroblasten aus dem umliegenden Gewebe können in das Fibrinnetz migrieren und es als provisorische Matrix nutzen. Es beginnt der Aufbau von Kollagenfasern. Durch das Enzym Plasmin kann das Fibringerüst mittels Fibrinolyse abgebaut werden. Die verschlossenen Gefäße können rekanalisiert werden.

Anders ausgedrückt, die Granulationsphase beginnt üblicherweise mit der Bildung von Granulationsgewebe. Hierfür ist im Allgemeinen eine beginnende Gefäßneubildung (Neoangiogenese) nötig, die sich bevorzugt durch eine sich vergrößernde Rotfärbung des Wundgewebes erkennen lässt. Gleichzeitig wird bevorzugt Matrixmaterial gebildet und das fehlende Gewebe mit gesunden Zellen aufgefüllt. Sobald das Granulationsgewebe seine physiologische Funktionsfähigkeit erreicht hat, kann üblicherweise die nächste Phase der Wundheilung beginnen.

Die Granulationsphase lässt sich folglich vorzugsweise durch die in Augenscheinnahme der Wunde erkennen. Die Wunde in dieser Phase zeigt ein auffällig rot gefärbtes, körniges (granuläres) Gewebe auf der Wundoberfläche, wobei diese Rotfärbung von anderen roten Komponenten, wie z.B. Blutkoageln, welche keine Zellen aufweisen, leicht zu unterscheiden ist, insbesondere für medizinisch geschultes Personal. Histologisch ist die Granulationsphase durch die Proliferation neuer kleiner Blutgefäße und die Einwanderung von Fibroblasten, deren Verbund zunehmend dichter wird, zu erkennen.

Der Fachmann (Mediziner) kann somit den Beginn und das Ende der Granulationsphase eindeutig abgrenzen. Zusätzlich sind zur Identifizierung der Granulationsphase weitere Methoden bekannt.

Weiterhin kann die Granulationsphase der Wundheilung durch die Bildung extrazellulärer Matrix, deren wichtigstes Protein Kollagen ist, charakterisiert werden. Kollagen weist normalerweise einen hohen Anteil an posttranslational gebildeten Hydroxyprolin auf. Damit kann die Kollagenbildung zur Bestimmung der Granulationsphase herangezogen werden.

In einer bevorzugten Ausführungsform wird daher die Granulationsphase durch Bestimmung des Kollagengehalt mittels C13-Methode durchgeführt. Mit $^{13}C$-Kohlenstoff markiertes Prolin wird für die Kollagensynthese verwendet

und anschließend zu Hydroxyprolin umgewandelt. Die Bestimmung von markiertem Hydroxyprolin in untersuchtem Gewebe stellt ein gutes Maß für die Kollagensynthese und somit für den Beginn der Granulationsphase dar. Nach der Methode von Babraj JA et al., Am J Physiol Endocrinol Metab: E864-E869, 2005 wird einer Untersuchungsperson eine Dosis von 0,75 g [1-$^{13}$C]-Prolin injiziert. Nach 2 Stunden werden eine Probe des zu untersuchenden Wundgewebes sowie eine gleich große Probe intakten Gewebes zum Vergleich entnommen. Es empfiehlt sich, die Vergleichsprobe von einer der Wunde entsprechenden Stelle zu entnehmen, bei einer Oberschenkelwunde z.B. von der der Wunde entsprechenden Stelle am nicht betroffenen zweiten Oberschenkel. Die Gewebeproben werden in flüssigem Stickstoff eingefroren, pulverisiert, mit 0,15 M NaCl-Lösung extrahiert, zentrifugiert, der Überstand wird abgetrennt. Das Pellet wird mit 70 %igem Ethanol gewaschen und erneut zentrifugiert. Das isolierte Pellet wird mit über Nacht sauer hydrolysiert. Die freigesetzten Amino- und Iminosäuren werden mittels Dowex 50W-X8 Ionenaustauscher getrennt. Die freigesetzten und getrennten Amino- und Iminosäuren werden als N-Acetyl-n-propylester derivatisiert und zur Messung des Einbaus von markiertem Prolin eingesetzt. Die Bestimmung von $^{13}$C-markiertem Hydroxyprolin erfolgt bevorzugt durch GC-MS über eine Kalibriergerade mit bekannten Hydroxyprolinkonzentrationen. Der Vergleich der Konzentrationen von $^{13}$C-markiertem Hydroxyprolin im Wundgewebe und im gesunden Gewebe zeigt, ob eine Kollagensynthese stattgefunden hat.

Eine alternative Methode ist die HPLC-Bestimmung von Glycin, Prolin und Hydroxyprolin nach der in EP 2 061 898 beschriebenen Methode. Diese Methode bestimmt den Kollagengehalt im Gewebe. Der Vergleich des Gewebekollagengehalts an zwei aufeinander folgenden Messzeitpunkten (z.B. zwei aufeinander folgenden Tagen) gibt Aufschluss darüber, wann neues Kollagen gebildet wird, wann also die Granulationsphase begonnen hat.

Im Rahmen dieser Erfindung wird die Granulationsphase bevorzugt durch in Augenscheinnahme des Fachmanns bestimmt. Sollte dies keine eindeutige Abgrenzung ermöglichen, wird bevorzugt die 13-C Bestimmung angewandt. Sollte auch hier keine eindeutige Abgrenzung möglich sein, dann wird die in EP 2 061 898 beschriebene Methode verwendet.

In einer bevorzugten Ausführungsform kann die Granulationsphase, insbesondere bei nicht chronischen Wunden, vom zweiten Tag bis zum zehnten Tag, bevorzugt vom zweiten bis zum achten Tag, mehr bevorzugt von dritten bis zum siebten Tag, insbesondere vom dritten bis zum sechsten Tag nach Auftreten der Wunde andauern.

In einer alternativen, bevorzugten Ausführungsform kann die Granulationsphase, insbesondere bei chronischen Wunden, von der zweiten Woche oder von der dritten Woche oder von vierten Woche bis zur dem Zeitpunkt dauern, an dem das Granulationsgewebe seine physiologische Funktionsfähigkeit erreicht hat, was mehrere Wochen oder gar Monate, zum Beispiel bis zu 6 Monate, dauern kann.

Mit der Differenzierungs- oder Umbauphase beginnt, etwa zwischen dem sechsten und zehnten Tag, üblicherweise die Ausreifung der kollagenen Fasern. Die Wunde kontrahiert sich durch die Umwandlung von Fibroblasten in Fibrozyten sowie Myofibroblasten. Dadurch schrumpft das Narbengewebe und es führt zu einer Verkleinerung der Wunde. Die Epithelisierung vom Wundrand her bringt die Wundheilung zum Abschluss.

In einer bevorzugten Ausführungsform ist die Behandlung (nicht beansprucht) der Wunden, bevorzugt der sekundär heilenden Wunden, eine phasengerechte Wundbehandlung. Unter einer phasengerechten Wundtherapie wird im Rahmen dieser Anmeldung verstanden, dass die Wundtherapie auf die spezifischen Bedürfnisse der Wunde in den einzelnen Phasen eingeht.

So kann die phasengerechte Behandlung gezielt in einer oder mehreren Phasen der Wundheilung erfolgen. (Im Gegensatz dazu erfolgt bei herkömmlichen Wundbehandlungen ein und dieselbe Behandlung über alle Phasen).

In einer bevorzugten Ausführungsform bedeutet "phasengerecht", dass die pharmazeutisch verträgliche Lösung, insbesondere Ringerlösung, gemäß der vorliegenden Erfindung bevorzugt in der Reinigungsphase und/oder in der Granulationsphase der Wunde, mehr bevorzugt in der Granulationsphase, insbesondere ausschließlich in der Granulationsphase, verwendet werden.

Ein weiterer Aspekt ist ein Verfahren zur Behandlung einer Wunde (nicht beansprucht) durch Anwenden der erfindungsgemäßen Wundauflage, insbesondere in der Granulationsphase.

Ein weiterer Aspekt (nicht beansprucht) ist eine erfindungsgemäße Wundauflage zur Verwendung in der Behandlung von Wunden, insbesondere in der Granulationsphase.

## Figuren

Nachstehend wird die erfindungsgemäße Wundauflage anhand von Zeichnungen näher erläutert. Die Erfindung soll jedoch nicht auf die in den Zeichnungen oder in der Beschreibung der Zeichnungen dargestellten Ausgestaltungen reduziert verstanden werden. Vielmehr umfasst die erfindungsgemäße Wundauflage auch Kombinationen der Einzelmerkmale der alternativen Formen.

Figur 1: schematischer Aufbau der erfindungsgemäßen Wundauflage
Figur 2: schematischer Aufbau einer Ausführungsform der erfindungsgemäßen Wundauflage
Figur 3: schematischer Aufbau einer weiteren Ausführungsform der erfindungsgemäßen Wundauflage

**Figurenlegende**

(a) Wundkontaktschicht
(b) hydrophile Schicht
(c) Trägerschicht
(d) Schicht umfassend einen Siloxan-enthaltenden Polyurethanschaumstoff oder einen Siloxan-funktionalisierten Polyurethanschaumstoff, welche auf der der wundabgewandten Seite der hydrophilen Schicht (b) angeordnet ist

In Figur 1 wird der schematische Aufbau der erfindungsgemäßen Wundauflage dargestellt. Die Wundauflage umfasst eine Wundkontaktschicht (a), welche den Siloxan-enthaltenden Polyurethanschaumstoff oder einen Siloxan-funktionalisierten Polyurethanschaum umfasst, und eine hydrophile Schicht (b), welche einen hydrophilen Schaumstoff umfasst. Die Wundkontaktschicht (a) ist im Wesentlichen flächig zu der hydrophilen Schicht (b) angeordnet. Die Wundauflage kann mithilfe von allgemein gebräuchlichen Schneidewerkzeugen der Größe und Form der Wunde angepasst werden. Folglich kann die Wundauflage in verschiedenen Abmessungen und Formen vorliegen, beispielsweise kreisförmig, oval oder rechteckig. Die Wundauflage kann aber auch in einer an die Wunde angepassten, unregelmäßigen Form vorliegen.

In Figur 2 wird der schematische Aufbau einer Ausführungsform der erfindungsgemäßen Wundauflage dargestellt. Die Wundauflage umfasst neben der Wundkontaktschicht (a), welche den Siloxan-enthaltenden Polyurethanschaumstoff oder einen Siloxan-funktionalisierten Polyurethanschaum umfasst, der hydrophilen Schicht (b), welche einen hydrophilen Schaumstoff umfasst, eine Trägerschicht (c). Die Wundkontaktschicht (a) und die hydrophile Schicht (b) sowie die hydrophile Schicht (b) und die Trägerschicht sind im Wesentlichen flächig zueinander angeordnet. In einer bevorzugten Ausführungsform umfasst die hydrophile Schicht (b) einen hydrophilen Polyurethanschaumstoff. In diesem Fall wird der Wundauflage durch die Trägerschicht zusätzliche Stabilität verliehen. Wie oben beschrieben, kann die Wundauflage kann mithilfe von allgemein gebräuchlichen Schneidewerkzeugen der Größe und Form der Wunde angepasst werden und damit in verschiedenen Abmessungen und regelmäßigen oder unregelmäßigen Formen vorliegen. Für den Fall, dass die Trägerschicht auch als Abdeckschicht verwendet wird (nicht in Figur 2 gezeigt), sollte die Größe des Trägerschicht (Abdeckschicht) so bemessen sein, dass die Trägerschicht außerhalb des Wundbereichs im Bereich der Wundumgebung befestigt werden kann.

In Figur 3 wird der schematische Aufbau einer weiteren Ausführungsform der erfindungsgemäßen Wundauflage dargestellt. Die Wundauflage umfasst neben der Wundkontaktschicht (a), welche den Siloxan-enthaltenden Polyurethanschaumstoff oder einen Siloxan-funktionalisierten Polyurethanschaum umfasst, der hydrophilen Schicht (b), welche einen hydrophilen Schaumstoff umfasst, eine Schicht (d), welche den Siloxan-enthaltenden Polyurethanschaumstoff oder einen Siloxan-funktionalisierten Polyurethanschaumstoff umfasst und auf der der wundabgewandten Seite der hydrophilen Schicht (b) angeordnet ist. Der in der Schicht (d) umfasste Siloxan-enthaltende Polyurethanschaumstoff oder Siloxan-funktionalisierte Polyurethanschaum ist vorzugsweise der gleiche Siloxan-enthaltende Polyurethanschaumstoff oder Siloxan-funktionalisierte Polyurethanschaumstoff wie in Wundkontaktschicht (a). Die Wundkontaktschicht (a) und die hydrophile Schicht (b) sowie die hydrophile Schicht (b) und die Schicht (d) sind im Wesentlichen flächig zueinander angeordnet. Durch diese symmetrische Sandwichstruktur ist die erfindungsgemäße Wundauflage beidseitig verwendbar, was die Anwendungssicherheit der Wundauflage signifikant erhöht. Weiterhin kann die Wundauflage in Wundkavitäten, bei denen Kontakt zu den gegenüberliegenden Oberflächen der Wundauflage mit dem Wundgrund zu erwarten sind, vorteilhat angewendet werden. Sie kann in die Wundkavitäten eingebracht werden, wobei durch die erwähnte "Sandwichstruktur" ein Verkleben mit dem Wundgrund auf beiden Seiten der Wundauflage vorteilhaft verhindert wird. Wie oben beschrieben, kann die Wundauflage mithilfe von allgemein gebräuchlichen Schneidewerkzeugen der Größe und Form der Wunde angepasst werden und damit in verschiedenen Abmessungen und regelmäßigen oder unregelmäßigen Formen vorliegen.

Ein hydrophiler Polyurethanschaum, der unter dem Namen Permafoam® der PAUL HARTMANN AG im Handel erhältlich ist wird auf der wundzugewandten Seite mit einem Siloxan-haltigen Schaum in einem Flammkaschierungsverfahren verbunden. Auf der wundabgewandten Seite des hydrophilen Polyurethanschaums wird der Verbund mit einem selbstklebenden Polyurethanfilm abgedeckt, wobei der Polyurethanfilm mit einem Druck von 200 N/ $m^2$ fest angedrückt wird.

## Patentansprüche

1. Wundauflage umfassend

(a) Wundkontaktschicht umfassend einen Siloxan-enthaltenden Polyurethanschaumstoff, wobei der Siloxan-enthaltende Polyurethanschaumstoff erhältlich ist durch Umsetzung einer härtbaren Mischung umfassend die Komponenten

(i) Polyisocyanat
(ii) Siloxan der Formel (A)

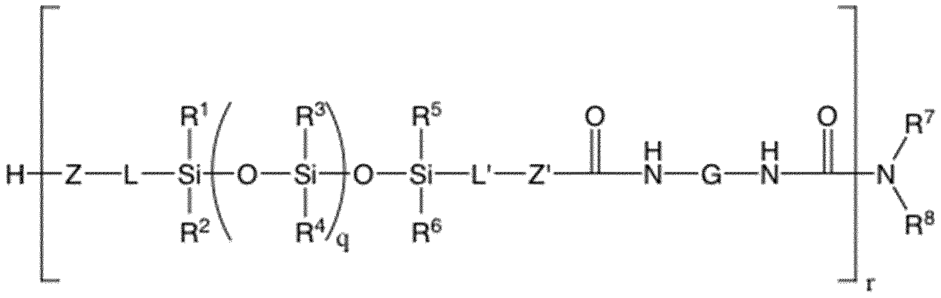

Formel (A),

wobei

$R^1$, $R^2$, $R^3$, $R^4$, $R^5$ und $R^6$ unabhängig voneinander Wasserstoff, ein substituierter oder ein unsubstituierter aliphatischer Kohlenwasserstoffrest mit 1 bis 8 C-Atomen oder ein substituierter oder ein unsubstituierter aromatischer Kohlenwasserstoffrest mit 6 bis 12 C-Atomen sind,
$R^7$ und $R^8$ unabhängig voneinander Wasserstoff, ein substituierter oder ein unsubstituierter aliphatischer Kohlenwasserstoffrest mit 1 bis 8 C-Atomen oder ein substituierter oder ein unsubstituierter aromatischer Kohlenwasserstoffrest mit 6 bis 12 C-Atomen sind, unter der Voraussetzung, dass mindestens einer der Reste $R^7$ und $R^8$ mindestens eine Hydroxyl- oder Thiolgruppe aufweist,
Z und Z' unabhängig voneinander Sauerstoff (O) oder Schwefel (S) sind,
L und L' unabhängig voneinander ein substituierter oder ein unsubstituierter aliphatischer Kohlenwasserstoffrest mit 1 bis 6 C-Atomen, wobei eines des C-Atome davon durch Sauerstoff (O) ersetzt sein kann, sodass eine Etherbindung vorliegt, oder ein substituierter oder unsubstituierter aromatischer Kohlenwasserstoffrest mit 6 bis 12 C-Atomen, welcher ein Heteroatom enthalten kann, sind,
G ein substituierter oder ein unsubstituierter aliphatischer Kohlenwasserstoffrest mit 1 bis 6 C-Atomen, oder ein substituierter oder unsubstituierter aromatischer Kohlenwasserstoffrest mit 6 bis 12 C-Atomen, welcher ein Heteroatom enthalten kann, ist,
q eine ganze Zahl von 1 bis 100 ist,
r eine ganze Zahl von 1 bis 10 ist, oder
einen Siloxan-funktionalisierten Polyurethanschaum,

(b) hydrophile Schicht umfassend einen hydrophilen Schaumstoff.

2. Wundauflage nach Anspruch 1, wobei der Siloxan-enthaltende Polyurethanschaumstoff ein offenzelliger, quervernetzter Siloxan-enthaltender Polyurethanschaumstoff ist.

3. Wundauflage nach Anspruch 1, wobei Z und Z' jeweils Sauerstoff (O) sind.

4. Wundauflage nach einem der vorangehenden Ansprüche, wobei der Siloxan-enthaltende Polyurethanschaumstoff eine Zugfestigkeit von 100 kPa bis 10 MPa und/oder eine Bruchdehnung von 100 % bis 350 %, jeweils gemessen gemäß DIN 53571, aufweist.

5. Wundauflage nach einem der vorangehenden Ansprüche, wobei der Siloxan-enthaltende Polyurethanschaumstoff eine Rohdichte von 10 und 120 kg/$m^3$ aufweist, gemessen nach DIN EN ISO 845:2009.

6. Wundauflage nach einem der vorangehenden Ansprüche, wobei die Wundkontaktschicht (a) eine Schichtdicke von 1 - 15 mm aufweist.

**7.** Wundauflage nach einem der vorangehenden Ansprüche, wobei die Wundkontaktschicht (a) Poren ausweist, vorzugweise Poren mit einem Durchmesser von 0.5 - 15 mm.

**8.** Wundauflage nach einem der vorangehenden Ansprüche, wobei die hydrophile Schicht (b) mit einer pharmazeutisch verträglichen Lösung, insbesondere Ringerlösung aktiviert ist.

**9.** Wundauflage nach einem der vorangehenden Ansprüche, wobei der hydrophile Schaumstoff ein Absorptionsvermögen von Kochsalzlösung zwischen 2 g/g und 30 g/g aufweist, gemessen nach DIN EN 13726-1.

**10.** Wundauflage nach einem der vorangehenden Ansprüche, wobei die hydrophile Schicht (b) einen hydrophilen Polyurethan- oder Polyvinylalkoholschaumstoff umfasst.

**11.** Wundauflage nach Anspruch 10, wobei der hydrophile Polyurethanschaumstoff ein Quellvermögen $\Delta V_1$ von höchstens 80 % aufweist.

**12.** Wundauflage nach einem der vorangehenden Ansprüche, weiter umfassend

(c) eine Trägerschicht und/oder
(d) eine Schicht umfassend einen Siloxan-enthaltenden Polyurethanschaumstoff oder einen Siloxan-funktionalisierten Polyurethanschaum, welche auf der der wundabgewandten Seite der hydrophilen Schicht (b) umfassend einen hydrophilen Schaumstoff angeordnet ist.

**13.** Verfahren zur Herstellung einer Wundauflage nach einem der vorangegangen Ansprüche, umfassend das Verbinden der Wundkontaktschicht (a) mit der hydrophilen Schicht (b), wobei das Verbinden der Schichten vorzugsweise Verkleben mit einem Adhäsiv, Ultraschallschweißen in einem kontinuierlichen Laminierprozess oder einen Thermobonding-Prozess umfasst.

**14.** Pharmazeutisch verträgliche Lösung, insbesondere Ringerlösung, zur Verwendung in der Behandlung von Wunden, insbesondere in der Granulationsphase, wobei die pharmazeutisch verträgliche Lösung in einer hydrophilen Schicht einer Wundauflage enthalten ist, wobei die Wundauflage umfasst:

(a) Wundkontaktschicht umfassend einen Siloxan-enthaltenden Polyurethanschaumstoff, wobei der Siloxan-enthaltende Polyurethanschaumstoff erhältlich ist durch Umsetzung einer härtbaren Mischung umfassend die Komponenten (i) Polyisocyanat (ii) Siloxan der Formel (A) gemäß Anspruch 1, wobei $R^1$, $R^2$, $R^3$, $R^4$, $R^5$ und $R^6$ unabhängig voneinander Wasserstoff, ein substituierter oder ein unsubstituierter aliphatischer Kohlenwasserstoffrest mit 1 bis 8 C-Atomen oder ein substituierter oder ein unsubstituierter aromatischer Kohlenwasserstoffrest mit 6 bis 12 C-Atomen sind,

$R^7$ und $R^8$ unabhängig voneinander Wasserstoff, ein substituierter oder ein unsubstituierter aliphatischer Kohlenwasserstoffrest mit 1 bis 8 C-Atomen oder ein substituierter oder ein unsubstituierter aromatischer Kohlenwasserstoffrest mit 6 bis 12 C-Atomen sind, unter der Voraussetzung, dass mindestens einer der Reste $R^7$ und $R^8$ mindestens eine Hydroxyl- oder Thiolgruppe aufweist,
Z und Z' unabhängig voneinander Sauerstoff (O) oder Schwefel (S) sind,
L und L' unabhängig voneinander ein substituierter oder ein unsubstituierter aliphatischer Kohlenwasserstoffrest mit 1 bis 6 C-Atomen, wobei eines des C-Atome davon durch Sauerstoff (O) ersetzt sein kann, sodass eine Etherbindung vorliegt, oder ein substituierter oder unsubstituierter aromatischer Kohlenwasserstoffrest mit 6 bis 12 C-Atomen, welcher ein Heteroatom enthalten kann, sind,
G ein substituierter oder ein unsubstituierter aliphatischer Kohlenwasserstoffrest mit 1 bis 6 C-Atomen, oder ein substituierter oder unsubstituierter aromatischer Kohlenwasserstoffrest mit 6 bis 12 C-Atomen, welcher ein Heteroatom enthalten kann, ist,
q eine ganze Zahl von 1 bis 100 ist,
r eine ganze Zahl von 1 bis 10 ist, oder ein Siloxan-funktionalisiertes Polyurethan,

(b) hydrophile Schicht umfassend einen hydrophilen Schaumstoff.

## Claims

1. A wound dressing comprising

(a) a wound contact layer comprising a siloxane-containing polyurethane foam, wherein the siloxane-containing polyurethane foam is obtainable by reacting a curable mixture comprising the following components

(i) a polyisocyanate
(ii) a siloxane of formula (A)

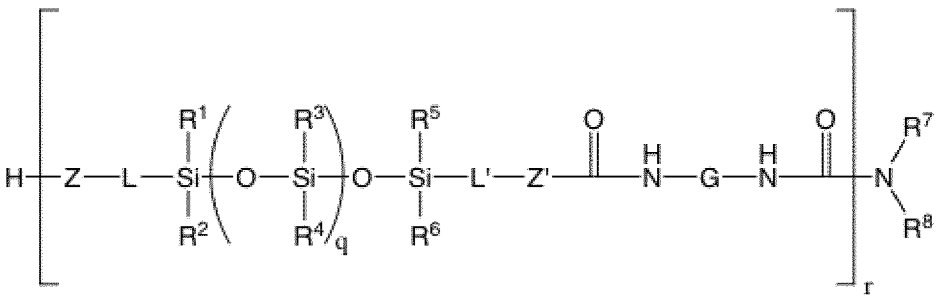

Formula (A),

wherein

$R^1$, $R^2$, $R^3$, $R^4$, $R^5$ and $R^6$ are each independently of one another hydrogen, a substituted or unsubstituted aliphatic hydrocarbon radical having from 1 to 8 carbon atoms or a substituted or unsubstituted aromatic hydrocarbon radical having from 6 to 12 carbon atoms,
$R^7$ and $R^8$ are each independently of one another hydrogen, a substituted or unsubstituted aliphatic hydrocarbon radical having from 1 to 8 carbon atoms or a substituted or unsubstituted aromatic hydrocarbon radical having from 6 to 12 carbon atoms, with the proviso that at least one of the radicals $R^7$ and $R^8$ has at least one hydroxyl or thiol group,
Z and Z' are each independently of one another oxygen (O) or sulfur (S), L and L' are each independently of one another a substituted or unsubstituted aliphatic hydrocarbon radical having from 1 to 6 carbon atoms, wherein one of the carbon atoms may be replaced by oxygen (O), so as to form an ether linkage, or a substituted or unsubstituted aromatic hydrocarbon radical having from 6 to 12 carbon atoms, which may contain a heteroatom,
G is a substituted or unsubstituted aliphatic hydrocarbon radical having from 1 to 6 carbon atoms, or a substituted or unsubstituted aromatic hydrocarbon radical having from 6 to 12 carbon atoms, which may contain a heteroatom,
q is an integer from 1 to 100,
r is an integer from 1 to 10, or
a siloxane-functionalized polyurethane foam,

(b) a hydrophilic layer comprising a hydrophilic foam.

2. The wound dressing according to claim 1, wherein the siloxane-containing polyurethane foam is an open-cell, cross-linked siloxane-containing polyurethane foam.

3. The wound dressing according to claim 1, wherein Z and Z' are each oxygen (O).

4. The wound dressing according to any one of the preceding claims, wherein the siloxane-containing polyurethane foam has a tensile strength of 100 kPa to 10 MPa and/or an elongation at break of 100% to 350%, each as measured according to DIN 53571.

5. The wound dressing according to any one of the preceding claims, wherein the siloxane-containing polyurethane foam has a bulk density of 10 and 120 kg/$m^3$, as measured according to DIN EN ISO 845:2009.

**6.** The wound dressing according to any one of the preceding claims, wherein the wound contact layer (a) has a layer thickness of 1 - 15 mm.

**7.** The wound dressing according to any one of the preceding claims, wherein the wound contact layer (a) comprises pores, preferably pores having a diameter of 0.5 - 15 mm.

**8.** The wound dressing according to any one of the preceding claims, wherein the hydrophilic layer (b) is activated with a pharmaceutically acceptable solution, in particular Ringer's solution.

**9.** The wound dressing according to any one of the preceding claims, wherein the hydrophilic foam has an absorption capacity of saline solution between 2 g/g and 30 g/g, as measured according to DIN EN 13726-1.

**10.** The wound dressing according to any one of the preceding claims, wherein the hydrophilic layer (b) comprises a hydrophilic polyurethane foam or polyvinyl alcohol foam.

**11.** The wound dressing according to claim 10, wherein the hydrophilic polyurethane foam has a swelling capacity $\Delta V_1$ of at most 80%.

**12.** The wound dressing according to any one of the preceding claims, further comprising

(c) a backing layer and/or
(d) a layer comprising a siloxane-containing polyurethane foam or a siloxane-functionalized polyurethane foam arranged on the side of the hydrophilic layer (b) comprising a hydrophilic foam facing away from the wound.

**13.** A method of manufacturing a wound dressing according to any one of the preceding claims, comprising bonding the wound contact layer (a) to the hydrophilic layer (b), wherein bonding the layers preferably comprises adhesion with an adhesive, ultrasonic welding in a continuous lamination process or a thermobonding process.

**14.** A pharmaceutically acceptable solution, in particular Ringer's solution, for use in the treatment of wounds, in particular in the granulation phase, wherein said pharmaceutically acceptable solution is contained in a hydrophilic layer of a wound dressing, wherein said wound dressing comprises:

(a) a wound contact layer comprising a siloxane-containing polyurethane foam, wherein the siloxane-containing polyurethane foam is obtainable by reacting a curable mixture comprising components (i) polyisocyanate (ii) siloxane of formula (A) according to claim 1, wherein $R^1$, $R^2$, $R^3$, $R^4$, $R^5$ and $R^6$ are each independently of one another hydrogen, a substituted or unsubstituted aliphatic hydrocarbon radical having from 1 to 8 carbon atoms or a substituted or unsubstituted aromatic hydrocarbon radical having from 6 to 12 carbon atoms,

$R^7$ and $R^8$ are each independently of one another hydrogen, a substituted or unsubstituted aliphatic hydrocarbon radical having from 1 to 8 carbon atoms or a substituted or unsubstituted aromatic hydrocarbon radical having from 6 to 12 carbon atoms, with the proviso that at least one of the radicals $R^7$ and $R^8$ has at least one hydroxyl or thiol group,
Z and Z' are each independently of one another oxygen (O) or sulfur (S), L and L' are each independently of one another a substituted or unsubstituted aliphatic hydrocarbon radical having from 1 to 6 carbon atoms, wherein one of the carbon atoms may be replaced by oxygen (O) so as to form an ether linkage, or a substituted or unsubstituted aromatic hydrocarbon radical having from 6 to 12 carbon atoms, which may contain a hetero atom,
G is a substituted or unsubstituted aliphatic hydrocarbon radical having from 1 to 6 carbon atoms, or a substituted or unsubstituted aromatic hydrocarbon radical having from 6 to 12 carbon atoms, which may contain a heteroatom,
q is an integer from 1 to 100,
r is an integer from 1 to 10, or
a siloxane-functionalized polyurethane,

(b) a hydrophilic layer comprising a hydrophilic foam.

## Revendications

1. Pansement comportant :

(a) une couche en contact avec la plaie comprenant une mousse de polyuréthane contenant du siloxane, dans lequel la mousse de polyuréthane contenant du siloxane peut être obtenue par conversion d'un mélange durcissable comprenant les composants

(i) polyisocyanate
(ii) siloxane de Formule (A)

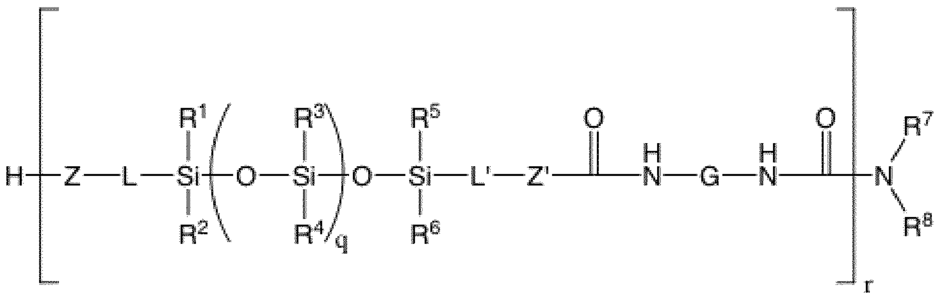

Formule (A)

dans laquelle

$R^1$, $R^2$, $R^3$, $R^4$, $R^5$ et $R^6$ sont, indépendamment les uns des autres, l'hydrogène, un radical hydrocarbure aliphatique substitué ou non substitué possédant 1 à 7 atomes de C ou un radical hydrocarbure aromatique substitué ou non substitué possédant 6 à 12 atomes de C,
$R^7$ et $R^8$ sont, indépendamment l'un de l'autre, l'hydrogène, un radical hydrocarbure aliphatique substitué ou non substitué possédant 1 à 8 atomes de C ou un radical hydrocarbure aromatique substitué ou non substitué possédant 6 à 12 atomes de C, à condition qu'au moins un des radicaux $R^7$ et $R^8$ comporte au moins un groupe hydroxyle ou thiol,
Z et Z' sont, indépendamment l'un de l'autre, l'oxygène (O) ou le soufre (S),
L et L' sont, indépendamment l'un de l'autre, un radical hydrocarbure aliphatique substitué ou non substitué possédant 1 à 6 atomes de C, dans lequel l'un des atomes de C de ceux-ci peut être remplacé par l'oxygène (O), de sorte qu'une liaison éther est présente, ou un radical hydrocarbure aromatique substitué ou non substitué possédant 6 à 12 atomes de C, qui peut contenir un hétéroatome,
G est un radical hydrocarbure aliphatique substitué ou non substitué possédant 1 à 6 atomes de C, ou un radical hydrocarbure aromatique substitué ou non substitué possédant 6 à 12 atomes de C, qui peut contenir un hétéroatome,
q est un nombre entier de 1 à 100,
r est un nombre entier de 1 à 10, ou
une mousse de polyuréthane fonctionnalisée par le siloxane,

(b) une couche hydrophile comprenant une mousse hydrophile

2. Pansement selon la revendication 1, dans lequel la mousse de polyuréthane contenant du siloxane est une mousse de polyuréthane contenant du siloxane réticulé transversalement, à alvéoles ouvertes.

3. Pansement selon la revendication 1, dans lequel Z et Z' sont chacun l'oxygène (O).

4. Pansement selon l'une des revendications précédentes, dans lequel la mousse de polyuréthane contenant du siloxane a une résistance à la traction de 100 kPa à 10 MPa et/ou un allongement à la rupture de 100 % à 350 %, respectivement mesurés conformément à la norme DIN 53571.

5. Pansement selon l'une des revendications précédentes, dans lequel la mousse de polyuréthane contenant du siloxane a une masse volumique apparente de 10 et 120 kg/$m^3$, mesurée conformément à la norme DIN EN ISO

845:2009.

6. Pansement selon l'une des revendications précédentes, dans lequel la couche en contact avec la plaie (a) a une épaisseur de couche de 1 à 15 mm.

7. Pansement selon l'une des revendications précédentes, dans lequel la couche en contact avec la plaie (a) comporte des pores, de préférence des pores ayant un diamètre de 0,5 à 15 mm.

8. Pansement selon l'une des revendications précédentes, dans lequel la couche hydrophile (b) est activée avec une solution pharmaceutiquement acceptable, en particulier une solution de Ringer.

9. Pansement selon l'une des revendications précédentes, dans lequel la mousse hydrophile a un pouvoir absorbant d'une solution saline entre 2 g/g et 30 g/g, mesuré conformément à la norme DIN EN 13726-1.

10. Pansement selon l'une des revendications précédentes, dans lequel la couche hydrophile (b) comprend une mousse de polyuréthane ou d'alcool polyvinylique hydrophile.

11. Pansement selon la revendication 10, dans lequel la mousse de polyuréthane hydrophile a un pouvoir de gonflement $\Delta V_1$ d'au moins 80 %.

12. Pansement selon l'une des revendications précédentes, comprenant en outre

(c) une couche de support et/ou
(d) une couche comprenant une mousse de polyuréthane contenant du siloxane ou une mousse de polyuréthane fonctionnalisée par le siloxane qui est agencée sur le côté de la couche hydrophile (b) opposé à la plaie, comprenant une mousse hydrophile.

13. Procédé pour fabriquer un pansement selon l'une des revendications précédentes, comportant l'assemblage de la couche en contact avec la plaie (a) avec la couche hydrophile (b), dans lequel l'assemblage des couches comprend de préférence un collage avec un adhésif, un soudage par ultrasons dans un processus de laminage continu ou un processus de liage thermique.

14. Solution pharmaceutiquement acceptable, en particulier une solution de Ringer, pour une utilisation dans le traitement de plaies, en particulier pendant la phase de granulation, dans laquelle la solution pharmaceutiquement acceptable est contenue dans une couche hydrophile d'un pansement, dans laquelle le pansement comprend :

(a) une couche en contact avec la plaie comprenant une mousse de polyuréthane contenant du siloxane, dans laquelle la mousse de polyuréthane contenant du siloxane peut être obtenue par conversion d'un mélange durcissable comprenant les composants (i) polyisocyanate (ii) siloxane de Formule (A) selon la revendication 1, dans laquelle $R^1$, $R^2$, $R^3$, $R^4$, $R^5$ et $R^6$ sont, indépendamment les uns des autres, l'hydrogène, un radical hydrocarbure aliphatique substitué ou non substitué possédant 1 à 8 atomes de C ou un radical hydrocarbure aromatique substitué ou non substitué possédant 6 à 12 atomes de C,

$R^7$ et $R^8$ sont, indépendamment l'un de l'autre, l'hydrogène, un radical hydrocarbure aliphatique substitué ou non substitué possédant 1 à 8 atomes de C ou un radical hydrocarbure aromatique substitué ou non substitué possédant 6 à 12 atomes de C, à condition qu'au moins un des radicaux $R^7$ et $R^8$ comporte au moins un groupe hydroxyle ou thiol,
Z et Z' sont, indépendamment l'un de l'autre, l'oxygène (O) ou le soufre (S),
L et L' sont, indépendamment l'un de l'autre, un radical hydrocarbure aliphatique substitué ou non substitué possédant 1 à 6 atomes de C, dans laquelle l'un des atomes de C de ceux-ci peut être remplacé par l'oxygène (O), de sorte qu'une liaison éther est présente, ou un radical hydrocarbure aromatique substitué ou non substitué possédant 6 à 12 atomes de C, qui peut contenir un hétéroatome,
G est un radical hydrocarbure aliphatique substitué ou non substitué possédant 1 à 6 atomes de C, ou un radical hydrocarbure aromatique substitué ou non substitué possédant 6 à 12 atomes de C, qui peut contenir un hétéroatome,
q est un nombre entier de 1 à 100,
r est un nombre entier de 1 à 10, ou un polyuréthane fonctionnalisé par le siloxane,

(b) une couche hydrophile comprenant une mousse hydrophile.

Figur 1:

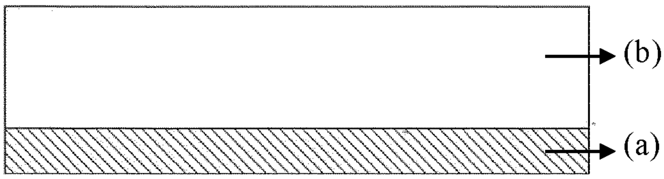


Figur 2:

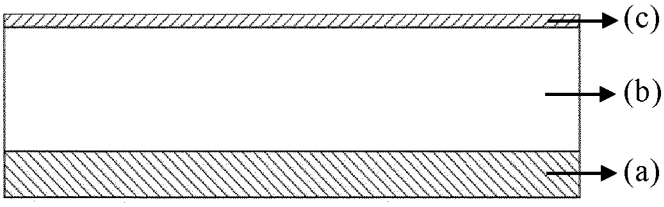


Figur 3:

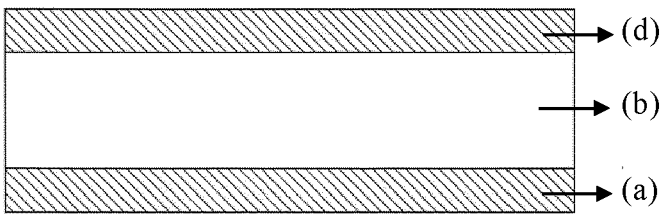

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2016156619 A1
- DE 102008031182
- EP 2061898 A


### In der Beschreibung aufgeführte Nicht-Patentliteratur


- **VON BABRAJ JA et al.** *Am J Physiol Endocrinol Metab,* 2005, E864-E869